(19) Europäisches Patentamt
European Patent Office
Office européen des brevets

(11) EP 0 549 900 B1

(12) **EUROPÄISCHE PATENTSCHRIFT**

(45) Veröffentlichungstag und Bekanntmachung des Hinweises auf die Patenterteilung:
**21.08.1996 Patentblatt 1996/34**

(51) Int. Cl.[6]: **C07F 17/00**, C08F 4/602

(21) Anmeldenummer: **92120287.5**

(22) Anmeldetag: **27.11.1992**

(54) **Metallocene mit benzokondensierten Indenylderivaten als Liganden, Verfahren zu ihrer Herstellung und ihre Verwendung als Katalysatoren**

Metallocenes containing benzocondensed indenyl derivatives as ligands, process for their preparation and their use as catalysts

Métallocènes contenant des dérivés d'indényl benzocondensé comme ligands, procédé pour leur préparation et leur application comme catalyseurs

(84) Benannte Vertragsstaaten:
**AT BE CH DE ES FR GB IT LI LU NL SE**

(30) Priorität: **30.11.1991 DE 4139595**

(43) Veröffentlichungstag der Anmeldung:
**07.07.1993 Patentblatt 1993/27**

(73) Patentinhaber: **HOECHST AKTIENGESELLSCHAFT**
**65926 Frankfurt am Main (DE)**

(72) Erfinder:
- **Rohrmann, Jürgen, Dr.**
  **W-6233 Kelkheim/Ts (DE)**
- **Dolle, Volker, Dr.**
  **W-6140 Bensheim (DE)**
- **Winter, Andreas, Dr.**
  **W-6246 Glashütten/Ts. (DE)**
- **Küber, Frank, Dr.**
  **W-6370 Oberursel (DE)**

(56) Entgegenhaltungen:
**EP-A- 0 185 918**
**EP-A- 0 320 762**
**EP-A- 0 426 643**

- **JOURNAL OF THE AMERICAN CHEMICAL SOCIETY Bd. 108, 1986, Seiten 179 - 181 SUDHAKAR, A. ET AL.**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 549 900 B1

Printed by Rank Xerox (UK) Business Services
2.13.2/3.4

## Beschreibung

Die vorliegende Erfindung betrifft neue Metallocene mit benzokondensierten Indenylderivaten als Liganden, die sehr vorteilhaft als Katalysatorkomponenten bei der Herstellung von Polyolefinen mit hoher Isotaktizität, enger Molmassenverteilung und hoher Molmasse verwendet werden können.

Polyolefine mit hoher Molmasse besitzen insbesondere Bedeutung für die Herstellung von Folien, Platten oder Großhohlkörpern wie z.B. Rohre oder Formteile.

Aus der Literatur ist die Herstellung von Polyolefinen mit löslichen Metallocenverbindungen in Kombination mit Aluminoxanen oder anderen Cokatalysatoren, die aufgrund ihrer Lewis-Acidität das neutrale Metallocen in ein Kation überführen und stabilisieren können, bekannt.

Beispielsweise wurde eine spezielle Voraktivierungsmethode des Metallocens mit einem Aluminoxan vorgeschlagen, welche zu einer beachtlichen Steigerung der Aktivität des Katalysatorsystems und zu einer deutlichen Verbesserung der Kornmorphologie des Polymeren führt (vgl. DE 37 26 067). Die Voraktivierung erhöht zwar die Molmasse, jedoch ist keine wesentliche Steigerung erreichbar.

Eine weitere, aber noch nicht ausreichende, Steigerung der Molmasse konnte durch Verwendung speziell heteroatomverbrückter Metallocene bei hoher Metallocenaktivität realisiert werden (EP-A 0 336 128).

Weiterhin sind Katalysatoren auf Basis Ethylenbisindenylhafniumdichlorid und Ethylen-bis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J.A. Ewen et al., J. Am.Chem.Soc. 109 (1987) 6544). Unter technisch relevanten Polymerisationsbedingungen ist jedoch die Kornmorphologie der derart erzeugten Polymeren nicht befriedigend und die Aktivität der eingesetzten Katalysatoren vergleichsweise gering. Verbunden mit den hohen Katalysatorkosten ist somit mit diesen Systemen eine kostengünstige Polymerisation nicht möglich.

Eine deutliche Steigerung der Molmasse konnte durch die Verwendung von Metallocenen erreicht werden, bei denen die durch eine Brücke fixierten aromatischen $\pi\Pi$-Liganden in 2-Stellung (DE-P 40 35 886.0) oder in 2- und 4-Stellung (DE-P 41 28 238.8) Substituenten tragen.

Unter dem Zwang großtechnisch kostengünstiger Produktion muß bei möglichst hohen Reaktionstemperaturen polymerisiert werden, da bei höheren Polymerisationstemperaturen die entstehende Polymerisationswärme mit weniger Kühlmedium abgeführt werden kann und daher mit einer deutlich geringeren Dimensionierung des Kühlwasserkreislaufes realisierbar ist.

Die letztgenannten Metallocene mit Substituenten in 2- bzw. 2- und 4-Stellung zur Brücke sind in dieser Hinsicht bei 70°C Polymerisationstemperatur bereits sehr leistungsfähig, trotzdem sind die erzielbaren Molmassen bei technisch relevanten Polymerisationstemperaturen (z.B. 70°C) für viele technische Anwendungen wie beispielsweise die Herstellung von Polymeren für Rohre und Großhohlkörper sowie spezielle Fasern noch zu gering.

In EP-A-519 237 werden Metallocen/Aluminoxan-Katalysatorsysteme beschrieben, welche sich zur Polymerisation von $C_2$- bis $C_{10}$-Alk-1-enen eignen. Als Metallocenkomponente wird u.a. die Verbindung Dimethylsilandiylbis(-2-methylbenzindenyl)-zirkoniumdichlorid genannt.

Es bestand die Aufgabe, ein Verfahren bzw. ein Katalysatorsystem zu finden, das Polymere mit guter Kornmorphologie und hoher Molmasse in großer Ausbeute erzeugt. Durch Verwendung von Wasserstoff als Molmassenregler kann dann die gesamte Molmassenpalette mit nur einem Metallocen abgedeckt werden.

Überraschenderweise wurde nun gefunden, daß Metallocene mit speziellen Indenylderivaten als Liganden geeignete Katalysatoren (Katalysatorkomponenten) bei der Herstellung von insbesondere isotaktischen Polyolefinen mit hoher Molmasse sind.

Gegenstand der vorliegenden Erfindung sind daher die Verbindungen der nachstehenden Formel I

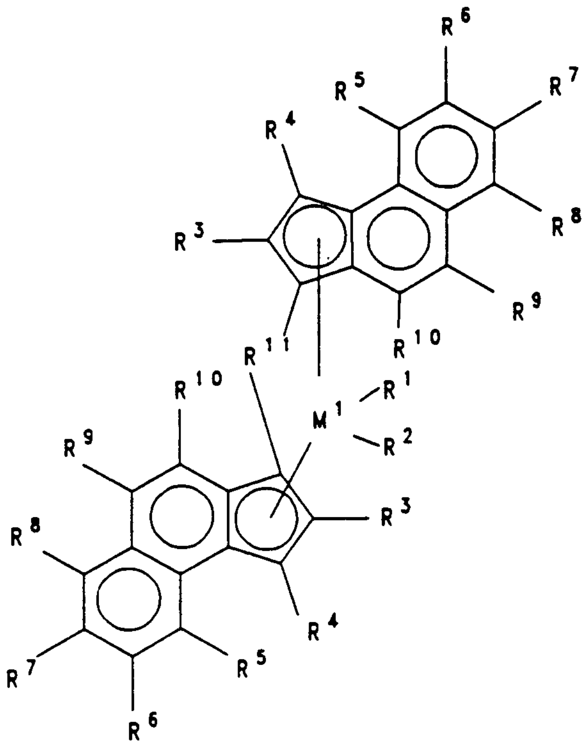

(I)

worin

| | |
|---|---|
| $M^1$ | ein Metall der Gruppe IVb, Vb oder VIb des Periodensystems ist, |
| $R^1$ und $R^2$ | gleich oder verschieden sind und ein Wasserstoffatom, eine $C_1$-$C_{10}$-Alkylgruppe, eine $C_1$-$C_{10}$-Alkoxygruppe, eine $C_6$-$C_{10}$-Arylgruppe, eine $C_6$-$C_{10}$-Aryloxygruppe, eine $C_2$-$C_{10}$-Alkenylgruppe, eine $C_7$-$C_{40}$-Arylalkylgruppe, eine $C_7$-$C_{40}$-Alkylarylgruppe, eine $C_8$-$C_{40}$-Arylalkenylgruppe, eine OH-Gruppe oder ein Halogenatom bedeuten, |
| die Reste $R^3$ | gleich oder verschieden sind und ein Wasserstoffatom, ein Halogenatom, eine $C_1$-$C_{10}$-Alkylgruppe, die halogeniert sein kann, eine $C_6$-$C_{10}$-Arylgruppe, einen $-NR_2$, -SR, $-OSiR_3$, $-SiR_3$ oder $-PR_2$-Rest bedeuten, worin R ein Halogenatom, eine $C_1$-$C_{10}$-Alkylgruppe oder eine $C_6$-$C_{10}$-Arylgruppe ist, |
| $R^4$ bis $R^{10}$ | die für $R^3$ genannten Bedeutungen besitzen, oder benachbarte Reste $R^4$ bis $R^{10}$ mit den sie verbindenden Atomen einen aromatischen oder aliphatischen Ring bilden, |
| $R^{11}$ | |

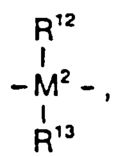 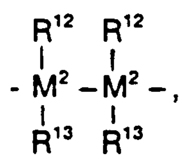 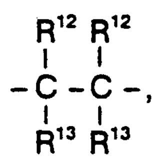 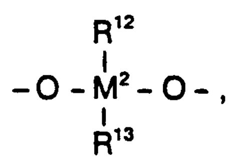

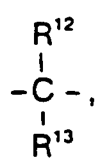 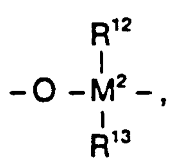 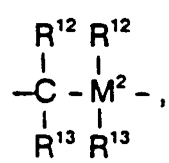

$=BR^{12}$, $=AlR^{12}$, -Ge-, -Sn-, -O-, -S-, =SO, $=SO_2$, $=NR^{12}$, =CO, $=PR^{12}$ oder $=P(O)R^{12}$ ist,
wobei

$R^{12}$ und $R^{13}$ gleich oder verschieden sind und ein Wasserstoffatom, ein Halogenatom, eine $C_1$-$C_{10}$-Alkylgruppe, $C_1$-$C_{10}$-Fluoralkylgruppe, eine $C_6$-$C_{10}$-Arylgruppe, eine $C_6$-$C_{10}$-Fluorarylgruppe, eine $C_1$-$C_{10}$-Alkoxygruppe, eine $C_2$-$C_{10}$-Alkenylgruppe, eine $C_7$-$C_{40}$-Arylalkylgruppe, eine $C_8$-$C_{40}$-Arylalkenylgruppe, eine $C_7$-$C_{40}$-Alkylarylgruppe bedeuten oder $R^{12}$ und $R^{13}$ jeweils mit den sie verbindenden Atomen einen Ring bilden und

$M^2$ Silizium, Germanium oder Zinn ist.

Alkyl steht für geradkettiges oder verzweigtes Alkyl, Halogen (halogeniert) bedeutet Fluor, Chlor, Brom oder Jod, bevorzugt Fluor oder Chlor.

Die Substituenten $R^4$ bis $R^{10}$ an den beiden Indenylliganden können trotz gleicher Bezeichnung verschieden sein (vgl. Definition von $R^3$).

In Formel I ist $M^1$ ein Metall der Gruppe IVb, Vb oder VIb des Periodensystems, beispielsweise Titan, Zirkon, Hafnium, Vanadium, Niob, Tantal, Chrom, Molybdän, Wolfram, vorzugsweise Zirkon, Hafnium und Titan.

$R^1$ und $R^2$ sind gleich oder verschieden und bedeuten ein Wasserstoffatom, eine $C_1$-$C_{10}$-, vorzugsweise $C_1$-$C_3$-Alkylgruppe, eine $C_1$-$C_{10}$-, vorzugsweise $C_1$-$C_3$-Alkoxygruppe, eine $C_6$-$C_{10}$-, vorzugsweise $C_6$-$C_8$-Arylgruppe, eine $C_6$-$C_{10}$-, vorzugsweise $C_6$-$C_8$-Aryloxygruppe, eine $C_2$-$C_{10}$-, vorzugsweise $C_2$-$C_4$-Alkenylgruppe, eine $C_7$-$C_{40}$-, vorzugsweise $C_7$-$C_{10}$-Arylalkylgruppe, eine $C_7$-$C_{40}$-, vorzugsweise $C_7$-$C_{12}$-Alkylarylgruppe, eine $C_8$-$C_{40}$-, vorzugsweise $C_8$-$C_{12}$-Arylalkenylgruppe oder ein Halogenatom, vorzugsweise Chlor.

Die Reste $R^3$ bis $R^{10}$ sind gleich oder verschieden und bedeuten ein Wasserstoffatom, ein Halogenatom, bevorzugt Fluor-, Chlor- oder Bromatom, eine $C_1$-$C_{10}$-, vorzugsweise $C_1$-$C_4$-Alkylgruppe, die halogeniert sein kann, eine $C_6$-$C_{10}$-, vorzugsweise $C_6$-$C_8$-Arylgruppe, einen $-NR_2$, -SR, $-OSiR_3$, $-SiR_3$ oder $-PR_2$-Rest, worin R ein Halogenatom, vorzugsweise Chloratom, oder eine $C_1$-$C_{10}$-, vorzugsweise $C_1$-$C_3$-Arylgruppe oder $C_6$-$C_{10}$-, vorzugsweise $C_6$-$C_8$-Arylgruppe ist.

$R^{11}$ ist

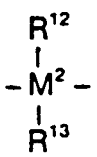 , 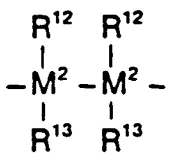 , 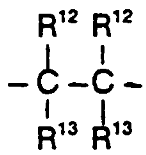 , 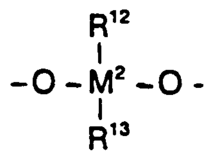 ,

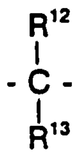 , 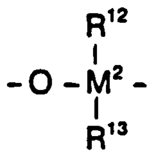 , 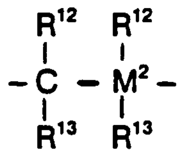 ,

$=BR^{12}$, $=AlR^{12}$, -Ge-, -Sn-, -O-, -S-, =SO, $=SO_2$, $=NR^{12}$, =CO, $=PR^{12}$ oder $=P(O)R^{12}$,

wobei $R^{12}$ und $R^{13}$ gleich oder verschieden sind und ein Wasserstoffatom, ein Halogenatom, eine $C_1$-$C_{10}$-, vorzugsweise $C_1$-$C_4$-Alkylgruppe, insbesondere Methylgruppe, eine $C_1$-$C_{10}$-Fluoralkylgruppe, vorzugsweise $CF_3$-Gruppe, eine $C_6$-$C_{10}$-, vorzugsweise $C_6$-$C_8$-Arylgruppe, eine $C_6$-$C_{10}$-Fluorarylgruppe, vorzugsweise Pentafluorphenylgruppe, eine $C_1$-$C_{10}$-, vorzugsweise $C_1$-$C_4$-Alkoxygruppe, insbesondere Methoxygruppe, eine $C_2$-$C_{10}$-, vorzugsweise $C_2$-$C_4$-Alkenylgruppe, eine $C_7$-$C_{40}$-, vorzugsweise $C_7$-$C_{10}$-Arylalkylgruppe, eine $C_8$-$C_{40}$-, vorzugsweise $C_8$-$C_{12}$-Arylalkenylgruppe oder eine $C_7$-$C_{40}$-, vorzugsweise $C_7$-$C_{12}$-Alkylarylgruppe bedeuten oder $R^{12}$ und $R^{13}$ bilden jeweils zusammen mit den sie verbindenden Atomen einen Ring.

$M^2$ ist Silizium, Germanium oder Zinn, bevorzugt Silizium und Germanium.

Für Verbindungen der Formel I gilt bevorzugt, daß

| | |
|---|---|
| $M^1$ | Zirkonium oder Hafnium, |
| $R^1$ und $R^2$ | gleich sind und eine $C_1$-$C_3$-Alkylgruppe oder ein Halogenatom, |
| die Reste $R^3$ | gleich sind und eine $C_1$-$C_4$-Alkylgruppe, |
| $R^4$ bis $R^{10}$ | gleich oder verschieden sind und Wasserstoff oder eine $C_1$-$C_4$-Alkylgruppe und |
| $R^{11}$ | |

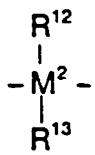 , 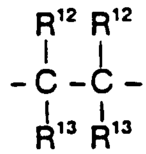 oder 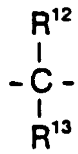 ,

wobei $M^2$ für Silizium und $R^{12}$ und $R^{13}$ gleich oder verschieden sind und für eine $C_1$-$C_4$-Alkylgruppe oder eine $C_6$-$C_{10}$-Arylgruppe stehen, bedeuten.

Weiterhin bevorzugt sind Verbindungen I, bei denen die Reste $R^4$ und $R^{10}$ Wasserstoff bedeuten und $R^5$ - $R^9$ für eine $C_1$-$C_4$-Alkylgruppe oder Wasserstoff stehen.

Insbesondere gilt, daß

| | |
|---|---|
| $M^1$ | Zirkonium, |
| $R^1$ und $R^2$ | gleich sind und Chlor, die Reste $R^3$ gleich sind und eine $C_1$-$C_4$-Alkylgruppe, $R^4$ und $R^{10}$ Wasserstoff, |
| $R^5$ bis $R^9$ | gleich oder verschieden sind und eine $C_1$-$C_4$-Alkylgruppe oder Wasserstoff und |
| $R^{11}$ | |

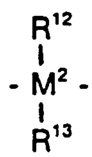 oder 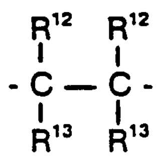 ,

wobei $M^2$ Silizium

und $R^{12}$ und $R^{13}$ gleich oder verschieden sind und eine $C_1$-$C_4$-Alkylgruppe oder eine $C_6$-$C_{10}$-Arylgruppe bedeuten, bedeuten.

Besonders bevorzugt sind die Verbindungen der Formel I, worin $M^1$ Zirkonium ist, $R^1$ und $R^2$ Chlor bedeuten, $R^3$ Methyl ist, $R^4$ bis $R^{10}$ Wasserstoff bedeuten und

$R^{11}$

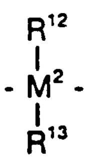

ist, wobei $M^2$ Silizium und $R^{12}$ und $R^{13}$

gleich oder verschieden sind und Methyl oder Phenyl bedeuten; insbesondere die in den Ausführungsbeispielen genannten Verbindungen I.

Gegenstand der vorliegenden Erfindung ist ferner ein Verfahren zur Herstellung einer Verbindung der Formel I, dadurch gekennzeichnet, daß man eine Verbindung der Formel IV

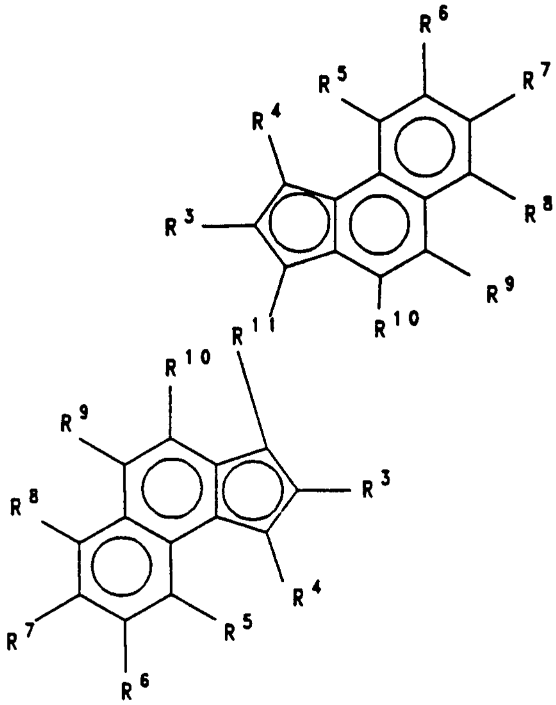

(IV)

wobei die Reste $R^3$ bis $R^{11}$ die in Formel I genannten Bedeutungen haben und $M^3$ ein Alkalimetall, bevorzugt Lithium, bedeutet, mit einer Verbindung der Formel V

$$M^1X_4 \qquad (V)$$

worin $M^1$ die in Formel I genannte Bedeutung besitzt und X ein Halogenatom, bevorzugt Chlor, bedeutet, umsetzt und das erhaltene Reaktionsprodukt gegebenenfalls derivatisiert.

Die Herstellung der Metallocene I erfolgt nach literaturbekannten Verfahren und ist im nachfolgenden Reaktionsschema wiedergegeben (vgl. ferner die Ausführungsbeispiele):

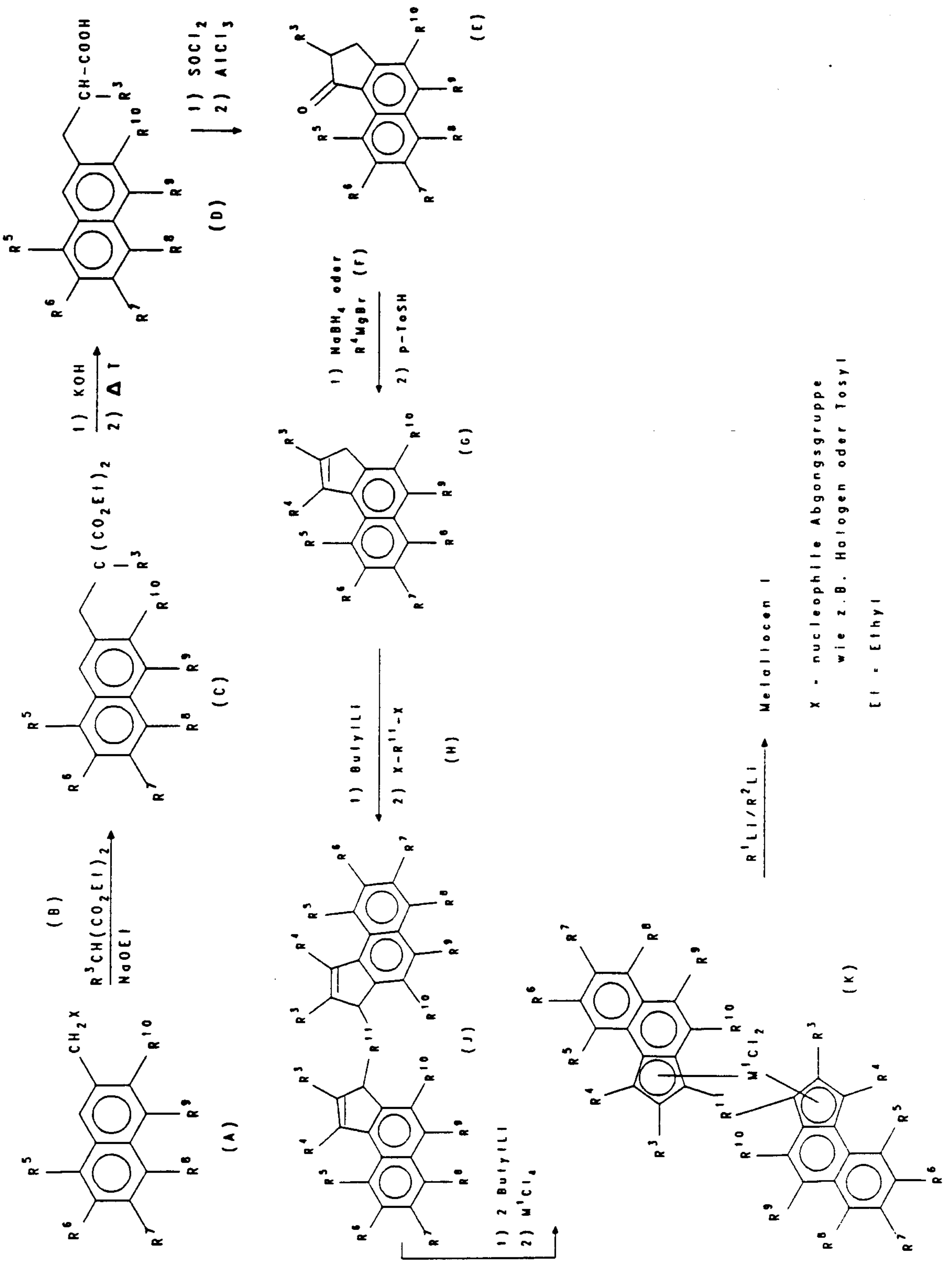


Die Naphthalinderivate der Formel A sind im Handel erhältlich oder können nach literaturbekannten Methoden hergestellt werden ("Friedel Crafts and Related Reactions", Wiley, New York, 1964, Vol. II, S. 659-766, Bull. Soc. Chim. Belges, 58 (1949) 87, J. Amer. Chem. Soc. 89 (1967) 2411).

Die Umsetzung zu den Verbindungen der Formel C erfolgt nach literaturbekannten Methoden durch Reaktion mit substituierten Malonsäureestern der Formel B unter basischen Bedingungen wie z.B. in ethanolischen Lösungen von Natriumethanolat (J. Org. Chem. 23 (1958) 1441, J. Am. Chem. Soc. 70 (1948) 3569).

Die Verbindungen der Formel C werden mit Alkalihydroxiden wie Kaliumhydroxid oder Natriumhydroxid nach literaturbekannten Methoden verseift und durch Thermolysieren der entstandenen Dicarbonsaure nach literaturbekannten Methoden zu den Verbindungen der Formel D decarboxyliert (J. Org. Chem. 23 (1958) 1441, J. Am. Chem. Soc. 70 (1948) 3569).

Der Ringschluß zu den substituierten Benzoindanonen der Formel E erfolgt nach literaturbekannten Methoden durch Umsetzung mit Chlorierungsreagentien wie z.B. $SOCl_2$ zu den entsprechenden Säurechloriden und anschließender Cyclisierung mit einem Friedel-Crafts-Katalysator in einem inerten Solvent, wie z.B. mit $AlCl_3$ oder Polyphosphorsäure in Methylenchlorid oder $CS_2$ (Organometallics 9 (1990) 3098, Bull. Soc. Chim. Fr. 3 (1967) 988, J. Org. Chem. 49 (1984) 4226).

Die Umsetzung zu den Benzoindenderivaten der Formel G ertolgt nach literaturbekannten Methoden durch Reduktion mit Natriumborhydrid oder Lithiumaluminiumhydrid in einem inerten Solvent wie z.B. Diethylether oder THF oder durch Alkylierung mit Alkylierungsmitteln der Formel F oder mit Lithiumalkylen zu den entsprechenden Alkoholen und Dehydratisierung der Alkohole unter sauren Bedingungen, wie z.B. mit p-Toluolsulfonsäure oder Oxalsäure oder durch Umsetzung mit wasserentziehenden Substanzen wie Magnesiumsulfat oder Molekularsiebe (Organometallics 9 (1990) 3098, Acta. Chem. Scand. B 30 (1976) 527, J. Amer. Chem. Soc. 65 (1943) 567).

Die Benzoindenderivate der Formel G können auch nach einer anderen, hier nicht näher aufgezeigten Syntheseroute ausgehend von substituierten Naphthalinen in 4 Syntheseschritten aufgebaut werden (Bull. Soc. Chim. Fr. 3 (1967) 988).

Die Herstellung der Ligandsysteme der Formel J und die Umsetzung zu den verbrückten chiralen Metallocenen der Formel K sowie die Isolierung der gewünschten racemischen Form ist im Prinzip bekannt (AU-A-31478/89, J. Organomet. Chem. 342 (1988) 21, EP 0 284 707, EP 0 320 762). Hierzu wird das Benzoindenderivat der Formel G mit starken Basen wie z.B. Butyllithium in einem inerten Lösemittel deprotoniert und mit einem Reagenz der Formel H zu dem Ligandsystem der Formel J umgesetzt. Dieses wird anschließend mit zwei Äquivalenten einer starken Base wie z.B. Butyllithium in einem inerten Lösemittel deprotoniert (Verbindung der Formel IV) und mit dem entsprechenden Metalltetrahalogenid wie z.B. Zirkontetrachlorid in einem geeigneten Lösemittel umgesetzt. Geeignete Lösemittel sind aliphatische oder aromatische Lösemittel, wie z.B. Hexan oder Toluol, etherische Lösemittel, wie z.B. Tetrahydrofuran oder Diethylether oder halogenierte Kohlenwasserstoffe, wie z.B. Methylenchlorid. Die Trennung der racemischen und der meso-Form erfolgt durch Extraktion oder Umkristallisation mit geeigneten Lösemitteln.

Die Derivatisierung zu den Metallocenen der Formel I kann nach literaturbekannten Methoden, z.B. durch Umsetzung mit Alkylierungsmitteln wie z.B. Methyllithium erfolgen (Organometallics 9 (1990) 1539, J. Amer. Chem. Soc. 95 (1973) 6263, EP 0 277 004).

Die erfindungsgemäßen Metallocene I sind hochaktive Katalysatorkomponenten für die Olefinpolymerisation.

Die vorliegende Erfindung umfaßt somit auch ein Verfahren zur Herstellung eines Olefinpolymers durch Polymerisation oder Copolymerisation eines Olefins der Formel $R^a$-CH=CH-$R^b$, worin $R^a$ and $R^b$ gleich oder verschieden sind und ein Wasserstoffatom oder einen Kohlenwasserstoffrest mit 1 bis 14 C-Atomen bedeuten, oder $R^a$ und $R^b$ mit den sie verbindenden Atomen einen Ring bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I ist.

Die chiralen Metallocene werden bevorzugt als Racemat eingesetzt. Verwendet werden kann aber auch die reine R- oder S-Form. Mit diesen reinen stereoisomeren Formen ist optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann. Wird die meso-Form nicht abgetrennt, entsteht neben isotaktischen Polymeren auch ataktisches Polymer. Für bestimmte Anwendungen - weiche Formkörper beispielsweise - kann dies durchaus wünschenswert sein.

Die Trennung der Stereoisomeren ist im Prinzip bekannt.

Erfindungsgemäß wird als Cokatalysator bevorzugt ein Alumninoxan der Formel (II)

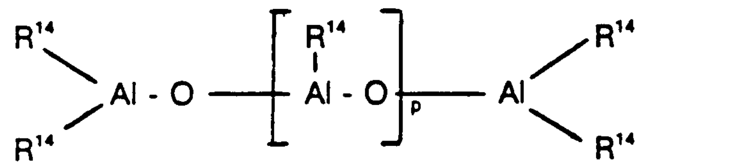

(II)

für den linearen Typ und/oder der Formel (III)

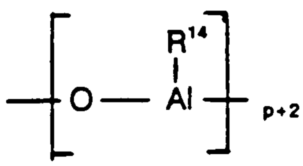

(III)

für den cyclischen Typ verwendet, wobei in den Formeln (II) und (III) die Reste $R^{14}$ gleich oder verschieden sein können und eine $C_1$-$C_6$-Alkylgruppe, eine $C_6$-$C_{18}$-Arylgruppe, Benzyl oder Wasserstoff bedeuten, und p eine ganze Zahl von 2 bis 50, bevorzugt 10 bis 35 bedeutet.

Bevorzugt sind die Reste $R^{14}$ gleich und bedeuten Methyl, Isobutyl, Phenyl oder Benzyl, besonders bevorzugt Methyl.

Sind die Reste $R^{14}$ unterschiedlich, so sind sie bevorzugt Methyl und Wasserstoff oder alternativ Methyl und Isobutyl, wobei Wasserstoff bzw. Isobutyl bevorzugt zu 0,01 - 40 % (Zahl der Reste $R^{14}$) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit veschiedenen Alkylgruppen $R^{14}$ werden entsprechend der gewünschten Zusammensetzung zwei verschiedene Aluminiumtrialkyle ($AlR_3$ + $AlR'_3$) mit Wasser umgesetzt (vgl. S. Pasynkiewicz, Polyhedron 9 (1990) 429 und EP-A 302 424).

Die genaue Struktur der Aluminoxane II und III ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel (II) und/oder (III) vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von $10^{-4}$ - 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polyolefinpulver in feinverteilter Form.

Erfindungsgemäß können an Stelle oder neben eines Aluminoxans Verbindungen der Formeln $R_xNH_{4-x}BR'_4$, $R_xPH_{4-x}BR'_4$, $R_3CBR'_4$ oder $BR'_3$ als geeignete Cokatalysatoren verwendet werden. In diesen Formeln bedeutet x eine Zahl von 1 bis 4, bevorzugt 3, die Reste R sind gleich oder verschieden, bevorzugt gleich, und bedeuten $C_1$-$C_{10}$-Alkyl, $C_6$-$C_{18}$-Aryl oder 2 Reste R bilden zusammen mit dem sie verbindenden Atom einen Ring, und die Reste R' sind gleich oder verschieden, bevorzugt gleich, und stehen für $C_6$-$C_{18}$-Aryl, das durch Alkyl, Haloalkyl oder Fluor substituiert sein kann.

Insbesondere steht R für Ethyl, Propyl, Butyl oder Phenyl und R' für Phenyl, Pentafluorphenyl, 3,5-Bistrifluormethylphenyl, Mesityl, Xylyl oder Tolyl (vgl. EP-A 277 003, EP-A 277 004 und EP-A 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösungsmittels hergestellt.

Prinzipiell ist als Cokatalysator erfindungsgemäß jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator bzw. das aus ihm gebildete Anion keine weiteren Reaktionen mit dem gebildeten Metallocenkation eingehen (vgl. EP-A 427 697).

Zur Entfernung von im Olefin vorhandener Katalystorgifte ist eine Reinigung mit einem Aluminiumalkyl, beispielsweise $AlMe_3$ oder $AlEt_3$ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase, kontinuierlich oder diskontinuierlich, ein- oder mehrstufig bei einer Temperatur von -60 bis 200°C, vorzugsweise 30 bis 80°C, besonders bevorzugt 50 bis 80°C, durchgeführt. Polymerisiert oder copolymerisiert werden Olefine der Formel $R^a$-CH=CH-$R^b$. In dieser Formel sind $R^a$ und $R^b$ gleich oder verschieden und bedeuten ein Wasserstoffatom oder einen Alkylrest mit 1 bis 14 C-Atomen. $R^a$ und $R^b$ können jedoch auch mit den sie verbindenden C-Atomen einen Ring bilden. Beispiele für solche Olefine sind Ethylen, Propylen, 1-Buten, 1-Hexen, 4-Methyl-1-penten, 1-Octen, Norbornen oder Norbornadien. Insbesondere werden Propylen und Ethylen polymerisiert.

Als Molmassenregler und/oder zur Aktivitätserhöhung wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von $10^{-3}$ bis $10^{-8}$, vorzugsweise $10^{-4}$ bis $10^{-7}$ mol Übergangsmetall pro $dm^3$ Lösemittel bzw. pro $dm^3$ Reaktorvolumen angewendet. Das Aluminoxan wird in einer Konzentration von $10^{-5}$ bis $10^{-1}$ mol, vorzugsweise $10^{-4}$ bis $10^{-2}$ mol pro $dm^3$ Lösemittel bzw. pro $dm^3$ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Pentan, Hexan, Heptan, Isooctan, Cyclohexan, Methylcyclohexan, genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivität zeigt.

Das erfindungsgemäße Verfahren zeichnet sich dadurch aus, daß die beschriebenen Metallocene im technisch besonders interessanten Temperaturbereich zwischen 50 und 80°C Polymere mit hoher Molmasse, hoher Stereospezifität und guter Kornmorphologie erzeugen.

Insbesondere die erfindungsgemäßen Zirkonocene stoßen in einen Molmassenbereich vor, oder übertreffen diesen sogar, der beim bisherigen Stand der Technik den Hafnocenen vorbehalten war. Diese hatten jedoch den Nachteil nur geringer Polymerisationsaktivität und sehr hoher Katalysatorkosten und die damit hergestellten Polymeren wiesen eine schlechte Pulvermorphologie auf.

Die nachfolgenden Beispiele sollen die Erfindung näher erläutern.

Es bedeuten:

VZ = Viskositätszahl in $cm^3/g$

| | | |
|---|---|---|
| $M_w$ = | Molmassengewichtsmittel in g/mol | ermittelt durch Gelpermeations-chromatographie |
| $M_w/M_n$ = | Molmassendispersität | |

Schmp. = Schmelzpunkt ermittelt mit DSC (20°C/min Aufheiz-/Abkühlgeschwindigkeit)

II = Isotakischer Index (II = mm+1/2 mr) ermittelt durch $^{13}$C-NMR_Spektroskopie

MFI/(230/5) Schmelzindex, gemessen nach DIN 53735; in dg/min

SD = Polymerschüttdichte in $g/dm^3$

Synthese der in den Polymerisationsbeispielen verwendeten Metallocene I:

Beispiel A

Synthese von rac-Dimethylsilandiylbis(2-methyl-4,5-benzoindenyl)-zirkondichlorid

1. Methyl(2-naphthylmethyl)malonsäurediethylester (1)

5,15 g (224 mmol) Natrium wurden in 150 ml absolutem Ethanol unter Erwärmen gelöst und bei Raumtemperatur mit 37,3 ml (217 mmol) Methylmalonsäurediethylester versetzt. Bei 0°C wurde eine Lösung von 50 g (217 mmol) 2-Brommethylnaphthalin (96 %) in 270 ml Ethanol langsam zugetropft und noch 4 bis 5 h zum Rückfluß erhitzt. Die Mischung wurde auf Eiswasser gegossen und mit Essigester extrahiert. Die vereinigten organischen Phasen wurden

mit Natriumsulfat getrocknet und eingedampft. Nach dem Trocknen im Ölpumpenvakuum wurde der ölige Rückstand bei 0°C mit Hexan verrührt, wobei 55 g (81 %) der Verbindung 1 kristallisierten.

2. 2-Methyl-3-naphthylpropionsäure (2)

33,2 g (105 mmol) der Verbindung 1 wurden in 70 ml Ethanol mit einer Lösung von 23,7 g (422 mmol) Kaliumhydroxid in 50 ml Wasser versetzt und 4 h zum Rückfluß erhitzt. Nach dem Abziehen des Lösemittels wurde der feste Rückstand in Essigester aufgenommen, mit Wasser versetzt und mit Salzsäure auf pH 1 eingestellt. Die wäßrige Phase wurde mehrmals mit Essigester extrahiert. Nach dem Trocknen über Magnesiumsulfat wurden die vereinigten organischen Phasen vollständig eingedampft. Der Rückstand wurde zur Kristallisation mit Hexan verrührt. Zur Decarboxylierung wurde der beigefarbene Feststoff bis zum Ende der Gasentwicklung auf 175°C erhitzt. Man erhielt 21 g (94 %) des Produktes 2 als beigefarbenen Feststoff.

3. 2-Methyl-6,7-benzoindan-1-on (3)

21 g (98 mmol) der Verbindung 2 wurden unter Feuchtigkeitsausschluß mit 22 ml Thionylchlorid versetzt und 30 min zum Rückfluß erhitzt. Anschließend wurde überschüssiges Thionylchlorid abdestilliert. Der Rückstand wurde kurz im Ölpumpenvakuum von flüchtigen Verbindungen befreit und dann in 25 ml Methylenchlorid unter Ar-Schutz gelöst. Die Lösung wurde bei Raumtemperatur langsam zu einer Suspension von 26 g (196 mmol) Aluminiumtrichlorid in 60 ml Methylenchlorid getropft und noch 30 min zum Rückfluß erhitzt. Die Mischung wurde auf Eis gegossen und mit Methylenchlorid extrahiert. Die vereinigten organischen Phasen wurden mit Natriumsulfat getrocknet und eingedampft. Der dunkle ölige Rückstand wurde an 600 g Kieselgel 60 chromatographiert. Mit einem Laufmittelgemisch aus Hexan/Essigester (9:3) konnten 8,6 g (45 %) der Verbindung 3 eluiert werden (gelblicher Feststoff).

4. 2-Methyl-4,5-benzoinden (4)

Eine Lösung von 7,8 g (39,7 mmol) des Indanons 3 in 400 ml einer THF/Methanol-Mischung (2:1) wurde bei Raumtemperatur portionsweise mit 2,2 g (59,5 mmol) Natriumborhydrid versetzt und 14 h gerührt. Die Lösung wurde auf HCl-saures Eis gegossen und ausgeethert. Die vereinigten organischen Phasen wurden mehrmals mit Wasser gewaschen und mit Natriumsulfat getrocknet. Das nach Abziehen des Lösemittels zurückgebliebene orangefarbene Öl wurde in 240 ml Toluol gelöst und mit 570 mg (3,15 mmol) p-Toluolsulfonsäure 15 min auf 80°C erhitzt. Die Lösung wurde bei Raumtemperatur mehrmals mit Wasser gewaschen, mit Natriumsulfat getrocknet und eingedampft. Der Rückstand wurde an 300 g Kieselgel 60 chromatographiert. Mit einem Laufmittelgemisch aus Hexan/Diisopropylether (20:1) konnten 4,7 g (65 %) des Indens 4 eluiert werden (farbloses Öl).
$^1$H-NMR-Spektrum (360 MHz, $CDCl_3$): 8,02 (1,d), 7,84 (1,m), 7,59 (1,d), 7,52 (1,d), 7,38-7,48 (2,m), 7,06 (1,m), 3,42 (2,s), 2,25 (3,d).

5. Dimethylbis(2-methyl-4,5-benzoindenyl)silan (5)

Eine Lösung von 4,6 g (25,5 mmol) des Indens 4 in 50 ml THF wurde bei Raumtemperatur mit 10,2 ml (25,5 mmol) einer 2,5 M Butyllithiumlösung in Hexan versetzt und 1 h zum Rückfluß erhitzt. Die rote Lösung wurde anschließend bei Raumtemperatur zu einer Lösung von 1,55 g (12 mmol) Dimethyldichlorsilan in 10 ml THF getropft und 5 bis 6 h zum Rückfluß erhitzt. Die Reaktionslösung wurde auf Eiswasser gegossen und mehrmals ausgeethert. Die mit Natriumsulfat getrockneten vereinigten organischen Phasen wurden eingedampft und im Ölpumpenvakuum getrocknet. Der Rückstand wurde an 300 g Kieselgel 60 chromatographiert. Mit einem Laufmittelgemisch aus Hexan/3 % Essigester konnten zunächst 500 mg nicht umgesetztes Edukt 4 eluiert werden. Mit dem gleichen Laufmittel folgte anschließend das Ligandsystem 5. Dieses konnte nach Abziehen des Lösemittels durch Verrühren mit Hexan kristallisiert werden (Isomere). Die Ausbeute betrug 1,7 g (34 % bzw. 44 % bezüglich umgesetztem Inden 4).

6. rac-Dimethylsilandiylbis(2-methyl-4,5-benzoindenyl)zirkondichlorid (6)

Eine Lösung von 1,7 g (4,1 mmol) des Ligangsystems 5 in 20 ml THF wurde bei Raumtemperatur unter Ar-Schutz mit 4,0 ml (10,2 mmol) einer 2,5 M Butyllithiumlösung in Hexan versetzt und 14 h bei Raumtemperatur gerührt. Der nach Abziehen des Lösemittels verbliebene Rückstand wurde im Ölpumpenvakuum getrocknet und mit Hexan gewaschen. Das so erhaltene hellbraune Pulver wurde mehrere Stunden im Ölpumpenvakuum bei 40 bis 50°C getrocknet und bei -78°C zu einer Suspension von 1,0 g (4,0 mmol) Zirkontetrachlorid in 25 ml Methylenchlorid gegeben. Nach dem Aufwärmen auf Raumtemperatur wurde das Lösemittel abgezogen und der Rückstand mit 20 ml Toluol extrahiert, um die meso-Form des Zirkonocens 6 zu entfernen. Der Rückstand des Toluolextraktes wurde anschließend mit 40 ml Methylenchlorid extrahiert. Die Lösung wurde auf ein kleines Volumen eingeengt und bei -35°C der Kristallisation überlassen. In mehreren Fraktionen konnten insgesamt 970 mg (42 %) des Zirkonocens 6 als reines Racemat isoliert werden.
$^1H$-NMR-Spektrum des Racemats (300 MHz, $CDCl_3$): 7,96 (2,m), 7,78 (2,m), 7,60 (2,d), 7,48-7,56 (4,m), 7,36 (2,d), 7,27 (2,s,β-Ind-H), 2,37 (6,s,Ind-$CH_3$), 1,36 (6,s,Si-$CH_3$). Massenspektrum: 574 $M^+$, korrekter Zerfall, korrektes Isotopenmuster.

Beispiel B

Synthese von rac-Dimethylsilandiyl bis(2-methyl-α-acenaphthindenyl)zirkondichlorid (10) (Nomenklatur analog Tebbe et al., J. Amer. Chem. Soc. 72 (1950) 3286)

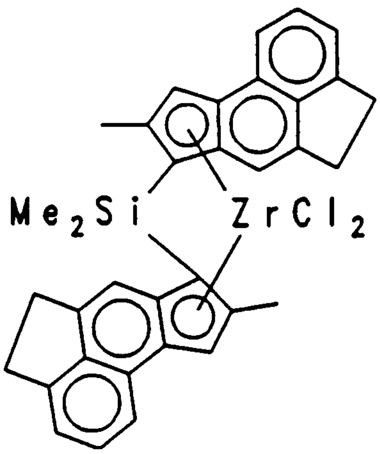

1. 2-Methyl-α-acenaphthindan-1-on (7)

Eine Lösung von 20 g (129 mmol) α-Acenaphthen in 320 ml Methylenchlorid wurde bei Raumtemperatur mit 29,7 g (129 mmol) 2-Bromisobuttersäurebromid versetzt. Anschließend wurden innerhalb 15 min über einen Feststoffdosiertrichter 43,5 g (324 mmol) $AlCl_3$ zugegeben. Nach 30 min Rühren wurde auf Eiswasser gegossen und mit Methylenchlorid extrahiert. Die organische Phase wurde mit Wasser und einer $NaHCO_3$-Lösung gewaschen und mit $NaSO_4$ getrocknet. Der nach Abziehen des Lösemittels verbliebene Rückstand wurde über eine kurze Säule mit Kieselgel filtriert. Mit Hexan/Essigester (9:2) erhielt man 25 g (87 %) des Indanons 7.
$^1H$-NMR ($CDCl_3$, 100 MHz): 8,57 (d,1), 7,60 (t,1), 7,35 d,1), 7,25 (s,1), 3,45 (q,1), 3,40 (s,4), 2,60-2,95 (m,2), 1,35 (d,3).

2. 2-Methyl-α-acenaphthinden (8)

Eine Lösung von 20 g (90 mmol) der Verbindung 7 in 250 ml einer THF/Methanol-Mischung (2:1) wurde zu einer Suspension von 3,8 g (100 mmol) $NaBH_4$ in 80 ml THF getropft. Die Mischung wurde 2 h bei Raumtemperatur gerührt und mit 100 ml Essigester und 100 ml halbkonz. HCl versetzt. Es wurde 10 min zum Rückfluß erhitzt und mit Essigester extrahiert. Die organische Phase wurde mit Wasser gewaschen und mit $NaSO_4$ getrocknet. Beim Einengen und Kühlen auf -35°C kristallisierten in mehreren Fraktionen insgesamt 16,3 g (88 %) der Verbindung 8.

3. Dimethylbis(2-methyl-α-acenaphthindenyl)silan (9)

10,8 g (52,4 mmol) der Verbindung 8 wurden analog Beispiel A/5 deprotoniert und mit Dimethyldichlorsilan umgesetzt. Die organische Phase wurde eingedampft und der Rückstand wurde an Kieselgel chromatographiert. Mit Hexan/4 % Essigester konnten 6,2 g (51 %) des Ligandsystems 9 gewonnen werden.
$^1H$-NMR ($CDCl_3$, 100 MHz): Diastereomerenpaar 7,1-7,8 (m,Aromaten-H),4,0 (s,CH), 3,45 (s,$CH_2$), 2,47 (d,$CH_3$), 2,40 (d,$CH_3$), -0,25 (s,$SiCH_3$), -0,35 (s,$SICH_3$), -0,37 (s,$SiCH_3$).

4. rac-Dimethylsilandiylbis(2-methyl-α-acenaphthindenyl)zirkondichlorid (10)

4,9 g (10,5 mmol) des Ligandsystems 9 wurden analog Beispiel A/6 umgesetzt. Das Rohprodukt, bestehend aus der racemischen mit der meso-Form im Verhältnis 1:1 wurde aus Chloroform mehrmals umkristallisiert. Man erhielt 1,3

g (20 %) des Racemats 10 in Form eines orangegelben Pulvers.
$^1$H-NMR ($CDCl_3$, 100 MHz): 7,0-7,8 (m,Aromaten-H), 3,1-3,4 (m,$CH_2$), 2,35 (s,$CH_3$), 1,35 (s,$SICH_3$)

Beispiel C

Synthese von rac-Methylphenylsilandiylbis(2-methyl-4,5-benzoindenyl)zirkondichlorid (12)

1. Methylphenylbis(2-methyl-2,5-benzoindenyl)silan (11)

Eine Lösung von 4,6 g (25,5 mmol) 2-Methyl-4,5-benzoinden (4, Beispiel A/4) in 50 ml THF wurde bei Raumtemperatur unter Ar-Schutz: mit 10,2 ml (25,5 mmol) einer 2,5 M Butyllithiumlösung in Hexan versetzt und 1 h zum Rückfluß erhitzt. Die rote Lösung wurde anschließend bei Raumtemperatur zu einer Lösung von 2,3 g (12 mmol) Methylphenyldichlorsilan in 10 ml THF getropft und 8 h zum Rückfluß erhitzt. Die Aufarbeitung und Reinigung erfolgte analog Beispiel A/5. Mit einem Laufmittelgemisch aus Hexan/5 % Essigester konnte zunächst nicht umgesetztes Edukt und anschließend 1,4 g (25 % bzgl. Si) des Ligandsystems 11 erhalten werden (Isomere).

2. rac-Methylphenylsilandiylbis(2-methyl-4,5-benzoindenyl)zirkondichlorid (12)

Eine Lösung von 1,3 g (2,71 mmol) des Liganden 11 in 15 ml THF wurde bei Raumtemperatur unter Ar-Schutz mit 1,2 ml (3 mmol) einer 2,5 M Butyllithiumlösung in Hexan versetzt und über Nacht bei Raumtemperatur gerührt. Das Lösemittel wurde abgezogen, der extrem luftempfindliche Rückstand mit Hexan gewaschen und mehrere Stunden im Ölpumpenvakuum getrocknet. Das Pulver wurde bei -78°C zu einer Suspension von 680 mg (2,9 mmol) $ZrCl_4$ in 15 ml $CH_2Cl_2$ gegeben. Nach dem langsamen Aufwärmen auf Raumtemperatur wurde noch 1 h bei dieser Temperatur gerührt und das Lösemittel abgezogen. Der Rückstand wurde zunächst mit wenig Toluol gewaschen und dann mit $CH_2Cl_2$ extrahiert. Beim Einengen und langsamen Abkühlen auf -35°C kristallisierten 380 mg (22 %) des Zirkonocens 12 als reines Racemat (orangegelbes Kristallpulver). Die nachfolgend auftretenden Mischfraktionen (Racemat und 2 meso-Formen) konnten durch mehrmaliges Umkristallisieren aus Chloroform oder Toluol gereinigt werden.
$^1$H-NMR-Spektrum des Racemats: (100 MHz, $CDCl_3$): 6,8-7,9 (m,Aromaten-H), 7,4 (s,β-Ind-H), 2,4 (s,Ind-$CH_3$), 2,1 (Ind-$CH_3$), 1,3 (s,Si-$CH_3$), Massenspektrum: 538 $M^+$, korrekter Zerfall, korrektes Isotopenmuster.

Beispiel D

Synthese von rac-Methylphenylsilandiylbis(2-methyl-α-acenaphthindenyl)zirkondichlorid (14)

1. Methylphenylbis(2-methyl-α-acenaphthindenyl)silan (13)

Eine Lösung von 10,8 g (52,4 mmol) 2-Methyl-α-acenaphthinden (8, Beispiel B/2) in THF wurde analog Beispiel A/5 mit 53 mmol Butyllithium und 4,9 g (26 mmol) Methylphenyldichlorsilan umgesetzt. Die Reaktionszeit betrug 12 h. Die Aufarbeitung erfolgte analog. Nach der Chromatographie mit Hexan/6 % Essigester erhielt man 6,0 g (44 %) des Ligandsstems 13 (Isomere).

2. rac-Methylphenylsilandiylbis(2-methyl-α-acenaphthindenyl)zirkondichlorid (14)

5,0 g (9,4 mmol) des Ligandsystems 13 wurden analog Beispiel A/6 mit 19,7 mmol Butyllithium und anschließend mit 2,2 g (9,4 mmol) $ZrCl_4$ umgesetzt und aufgearbeitet. Der Rückstand wurde zur Entfernung der meso-Formen mehrmals aus Methylenchlorid umkristallisiert. Man erhielt 1,2 g (19 %) des Metallocens 14 als reines Racemat in Form eines orangegelben Pulvers.
$^1$H-NMR ($CDCl_3$, 100 MHz): 6,8-7,8 (m,Aromaten-H), 3,0-3,4 (m,$CH_2$), 2,4 (s,$CH_3$), 2,1 (s,$CH_3$), 1,3 (s,$SiCH_3$). Massenspektrum: 690 $M^+$, korrekter Zerfall, korrektes Isotopenmuster.

Beispiel E

Synthese rac-1,2-Ethandiylbis(2-methyl-4,5-benzoindenyl)zirkondichlorid (15)

1. 1,2-Bis(2-methyl-4,5-benzoindenyl)ethan (14)

Eine Lösung von 18,0 g (100 mmol) 2-Methyl-4,5-benzoinden 4 (Beispiel A/4) in 400 ml THF wurde bei Raumtemperatur mit 40 ml (100 mmol) einer 2,5 M Butyllithiumlösung in Hexan versetzt und 30 min zum Rückfluß erhitzt. Bei -78°C wurden 9,35 g (50 mmol) 1,2-Dibromethan zugegeben. Die Mischung wurde über Nacht auf Raumtemperatur

erwärmt, auf salzsaures Eiswasser gegossen und mit Diethylether extrahiert. Nach dem Waschen mit $NaHCO_3$-Lösung und Trocknung mit $MgSO_4$ wurde das Lösemittel abgezogen und der Rückstand an Kieselgel 60 chromatographiert. Mit Hexan/6 % Essigester ließen sich nach nicht umgesetztem Edukt und einem Nebenprodukt (Spiroverbindung) 8,6 g (45 %) des Ligandsystems 14 eluieren.

2. rac-1,2-Ethandiylbis(2-methyl-4,5-benzoindenyl)zirkondichlorid (15)

Eine Lösung von 4,2 g (10,8 mmol) des Liganden 14 wurden analog Beispiel A/6 mit Butyllithium und $ZrCl_4$ umgesetzt. Die Extraktion des Rückstandes mit Methylenchlorid/Toluol und Kristallisation bei -35°C lieferte 1,4 g (24 %) des Metallocens als reines Racemat in Form eines gelben mikrokristallinen Pulvers.
$^1$H-NMR-Spektrum (100 MHz, $CDCl_3$): 7,3-8,0 (m,Aromaten-H), 7,1 (s,β-H) 3,4-4,1 (m,$CH_2CH_2$), 2,2 (s,$CH_3$).
Massenspektrum: 546 $M^+$, korrektes Isotopenmuster, korrekter Zerfall.

Beispiel F

Synthese rac-1,2-butandiylbis(2-methyl-4,5-benzoindenyl)zirkondichlorid (17)

1. 1,2-Bis(2-methyl-4,5-benzoindenyl)butan (16)

18,0 g (100 mmol) 2-Methyl-4,5-benzoinden (4, Beispiel A/4) wurden analog Beispiel E/1 mit 10,7 g (50 mmol) 1,2-Dibrombutan (97 %) umgesetzt und aufgearbeitet. Die Chromatographie an Kieselgel 60 mit Hexan/2 % Essigester lieferte nach nicht umgesetztem Edukt und der Spiroverbindung 3,9 g (19 %) des Ligandsystems 16 als Isomerengemisch. In einer nachfolgenden Chromatographie an einer langen Säule konnte mit einem Laufmittelgemisch aus Hexan und anschließend Hexan/1-3 % Essigester die einzelnen Isomere getrennt bzw. angereichert werden.

2. rac-1,2-Butandiylbis(2-methyl-4,5-benzoindenyl)zirkondichlorid (17)

1,0 g (2,41 mmol) des Liganden 16 (2 Isomere) wurden analog Beispiel A/6 mit Butyllithium und $ZrCl_4$ umgesetzt. Die Extraktion mit Toluol/Methylenchlorid (5:1) und langsame Kristallisation durch Einengen und Abkühlen auf -35°C lieferte insgesamt 0,89 g (65 %) Kristallfraktionen des Metallocens 17 unterschiedlicher Zusammensetzung der verschiedenen Isomere der rac- und meso-Formen (durch zusätzliches Chiralitätszentrum an der Brücke). Bei nochmaliger Umkristallisation konnte eine Fraktion des Racemats 17 (Diastereomerenpaar) gewonnen werden.
Massenspektrum: 574 $M^+$, korrektes Isotopenmuster, korrekter Zerfall.

Beispiel G

Synthese rac-Dimethylsilandiylbis(4,5-benzoindenyl)zirkondichlorid (23)

1. 2-Naphthylmethylmalonsäurediethylester (18)

34,7 g (217 mmol) Malonsäurediethylester wurden analog Beispiel A/1 umgesetzt und aufgearbeitet. Beim Behandeln mit Hexan erhielt man 87 g eines bräunlichen Öls der Verbindung 18.

2. 3-Naphthylpropionsäure (19)

87 g der Verbindung 18 wurden analog Beispiel A/2 mit KOH behandelt und thermolysiert. Man erhielt 36 g (83%) der Verbindung 19 als beigefarbenes Pulver.

3. 6,7-Benzoindan-1-on (20)

33,6 g (168 mmol) der Verbindung 19 wurden analog Beispiel A/3 mit $SOCl_2$ und $AlCl_3$ umgesetzt. Die Reaktionszeit der Cyclisierung betrug 15 min bei 40°C. Man erhielt nach Chromatographie (teilweise Zersetzung auf der Säule) 9,4 g (30 %) des Indanons 20 als gelblichen Feststoff (teilweise ölig).
$^1$H-NMR-Spektrum (100 MHz, $CDCl_3$): 9,15 (dd,1,Aromaten-H), 7,35-81, (m,5,Aromaten-H), 3,2 (m,2,$CH_2$), 2,80 (m,$CH_2$).

4. 4,5-Benzoinden (21)

9,4 g (51,6 mmol) des Indanons 20 wurden analog Beispiel A/4 reduziert. Die Dehydratisierung erfolgte in einer Destillationsapparatur unter Zusatz von 6 g $MgSO_4$. Bei 110°C und 0,6-0,9 mbar gingen 2,6 g (30 %) des Indens 21 in Form eines farblosen Destillats über, das bei Raumtemperatur erstarrte.
$^1$H-NMR-Spektrum (100 MHz, $CDCl_3$): 7,35-8,2 (m,7,Aromaten-H und CH), 6,70 (dt,1,CH), 3,55 (t,$CH_2$).

5. Dimethylbis(4,5-benzoindenyl)silan (22)

3,25 g (19,6 mmol) des Indens 21 wurden analog Beispiel A/5 umgesetzt. Die Chromatographie an 600 g Kieselgel 60 lieferte mit Hexan und Hexan/Essigester 20:1 neben Edukt 1,8 g (47 %) des Ligandsystems 22 (Isomere).
$^1$H-NMR-Spektrum (100 MHz, $CDCl_3$): 7,3-8,2 (m,Aromaten-H), 6,6-6,9 (m,CH), 3,5-4,1 (m,CH), -0,35-0,20 (mehrere Sing.,$SiCH_3$).

6. rac-Dimethylsilandiylbis(4,5-benzoindenyl)zirkondichlorid (23)

1,6 g (4,12 mmol) des Liganden 22 wurden analog Beispiel A/6 mit Butyllithium und $ZrCl_4$ umgesetzt. Nach der Extraktion mit Methylenchlorid ließen sich bei -35°C 520 mg (23 %) des Metallocens 23 als Racemat isolieren (gelb-oranges Pulver).
$^1$H-NMR-Spektrum (100 MHz,$CDCl_3$): 7,2-8,0 (m,Aromaten-H), 7,2 (d,$\beta$-CH), 6,4 (d,$\alpha$-CH), 1,2 (s,$SiCH_3$).

Polymerisationsbeispiele

Beispiele 1 und 2

Ein trockener 16-$dm^3$-Reaktor wurde mit Stickstoff gespült und mit 10 $dm^3$ flüssigem Propylen befüllt. Dann wurden zwei Drittel der in der Tabelle 1 angegebenen MAO-Menge als toluolische Lösung zugesetzt und der Ansatz 15 min bei 30°C gerührt.

Parallel dazu wurde eine toluolische Lösung des Metallocens Dimethylsilylbis(2-methyl-4,5-benzoindenyl)zirkondichlorid in einem Drittel der angegebenen MAO-Menge hergestellt und durch 15 minütiges Stehenlassen voraktiviert. Die Lösung kann auch unter Rühren oder Schütteln oder im Ultraschallbad voraktiviert werden. Diese Lösung wurde dann in den Reaktor gegeben und die Polymerisation wurde durch Erwärmen auf die Solltemperatur gestartet. Der Ansatz wurde durch Abkühlen und Entspannen nach einer Stunde gestoppt. Die erhaltene Polymerausbeute und die ermittelten analytischen Daten sind der Tabelle 1 zu entnehmen.

Tabelle 1:

| Bsp. | Temp. [°C] | Metallocen-menge [mg] | MAO-Menge [mmol] | Ausbeute [kg] | Aktivität [kg PP/g Metallocen x h] | VZ [$cm^3$/g] | MFI/ (230/5) [dg/min] |
|---|---|---|---|---|---|---|---|
| 1 | 70 | 5,2 | 60 | 2,24 | 431 | 288 | 5,6 |
| 2 | 50 | 7,3 | 60 | 1,72 | 235 | 444 | 2,4 |

| $M_w/M_n$ | $M_w$ [g/mol] | Schmp. [°C] |
|---|---|---|
| 1,8 | 330 000 | 147 |
| 2,0 | 540 000 | 149 |

MAO = Methylaluminoxan

PP = Polypropylen

Beispiele 3 und 4

Ein trockener 24 $dm^3$-Reaktor wurde mit Propylen gespült, evakuiert und mit 12 $dm^3$ flüssigem Propylen befüllt. Dann wurden 25 $cm^3$ toluolische Methylaluminoxanlösung (entsprechend 37 mmol Al, mittlerer Oligomerisierungsgrad p = 18) zugegeben und der Ansatz bei 30°C 15 Minuten gerührt.

Parallel dazu wurden die in der Tabelle 2 angegebenen Mengen des Metallocens rac-Dimethylsilandiylbis(2-methyl-$\alpha$-acenaphthindenyl)$ZrCl_2$ in 10 $cm^3$ toluolischer Methylaluminoxanlösung (15 mmol Al) gelöst, entsprechend Beispiel 1 voraktiviert, und in den Polymerisationen eingesetzt. Die Polymerisationen wurden ebenfalls wie in Beispiel 1 beschrieben durchgeführt. Einzelheiten der Polymerisation und Polymerausprüfung sind der Tabelle 2 zu entnehmen.

Tabelle 2

| Bsp. | mg Metallocen | Polymerisations-temp. (°C) | Polymer-dauer (h) | Metallocen-aktivität (kg PP/g Metallocen x h) | VZ ($cm^3/g$) | MFI (230/5) (dg/min) |
|---|---|---|---|---|---|---|
| 3 | 4,2 | 70 | 1 | 285 | 239 | 16,0 |
| 4 | 7,1 | 50 | 1,5 | 69,4 | 452 | 1,1 |

| Bsp. | $M_w$ (g/mol) | $M_w/M_n$ | Schmp. (°C) |
|---|---|---|---|
| 3 | 248 500 | 2,0 | 149 |
| 4 | 521 000 | 2,1 | 150 |

Beispiele 5 bis 9

Beispiel 3 wurde wiederholt. Es wurden jedoch die in Tabelle 3 aufgeführten Metallocene verwendet. Die Ergebnisse der Polymerisationen sind ebenfalls der Tabelle 3 zu entnehmen.

Beispiel 10

Es wurde verfahren wie in Beispiel 3, verwendet wurden jedoch nur 2,6 mg des Metallocens und zusätzlich wurden 2,5 $Ndm^3$ Wasserstoff in den Reaktor gegeben.

Die Metallocenaktivität war 496 kg PP/g Metallocen x h, VZ = 187 $cm^3/g$, MFI (230/5) = 28,5 dg/min, Schmp. = 151°C.

Beispiel 11

Es wurde verfahren wie in Beispiel 10, die Wasserstoffmenge war jedoch 25 $Ndm^3$. Die Metallocenaktivität war 598 kg PP/g Metallocen x h, VZ = 105 $cm^3/g$, Schmp. 149°C.

| Beispiel | Metallocen | Metallocenaktivität [kg PP/g Metallocen x h] | VZ [$cm^3/g$] | MFI (230/5) [dg/min] | $M_w$ [g/mol] | $M_w/M_n$ | Schmp. [°C] |
|---|---|---|---|---|---|---|---|
| 5 | 3,5 mg rac-Ph(Me)Si bis-(2-methyl-4,5-benzoindenyl)-zirkondichlorid | 236 | 355 | 3,1 | 431 500 | 2,2 | 150 |
| 6 | 4,7 mg rac-Ph(Me)Si bis-(2-methyl-α-acenaphthindenyl)-zirkondichlorid | 218 | 298 | 4,5 | 330 000 | 2,0 | 148 |
| 7 | 3,0 mg rac-1,2-Ethandiylbis-(2-methyl-4,5-benzoindenyl)-zirkondichlorid | 505 | 198 | 26,5 | 219 000 | 2,3 | 145 |
| 8 | 4,3 mg rac-1,2-Butandiylbis-(2-methyl-4,5-benzoindenyl)-zirkondichlorid | 422 | 279 | 5,7 | 321 000 | 2,1 | 146 |
| 9 | 2,8 mg rac-$Me_2$Si bis-(4,5-benzoindenyl)zirkon-dichlorid | 438 | 117 | 205 | 124 500 | 2,0 | 141 |

Beispiel 12

Es wurde verfahren wie in Beispiel 3, verwendet wurden jedoch 2,8 mg des Metallocens rac-Dimethylsilandiylbis(2-methyl-4,5-benzoindenyl)zirkondichlorid und die Wasserstoffmenge war 15 $Ndm^3$.
Die Metallocenaktivität war 647 kg PP/g Metallocen x h, VZ = 147 $cm^3/g$, Schmp. = 148°C.

Die Beispiele 10 bis 12 belegen die gute Wasserstoffansprechbarkeit zur Molmassenregelung bei den erfindinngsgemäßen Metallocenen.

Beispiel 13

Ein trockener 24 $dm^3$-Reaktor wurde mit Propylen gespült und mit 2,4 $Ndm^3$ Wasserstoff und 12 $dm^3$ flüssigem Propylen befüllt. Dann wurden 35 $cm^3$ toluolische Methylaluminoxanlösung (entsprechend 52 mmol Al, mittlerer Oligomerisierungsgrad p = 19) zugegeben. Parallel dazu wurden 3,9 mg rac-Dimethyl-silandiylbis(2-methyl-4,5-benzoindenyl)zirkondichlorid in 13,5 $cm^3$ toluolischer Methylaluminoxanlösung (20 mmol Al) gelöst und durch 5 minütiges stehenlassen voraktiviert.
Die Lösung wird dann in den Reaktor gegeben und unter kontinuierlicher Zugabe von 100 g Ethylen wurde 1 h bei 60°C polymerisiert. Die Metallocenaktivität betrug 409 kg PP/g Metallocen x h, der Ethylengehalt des statistischen Copolymers betrug 5,7 Gew.-%.
VZ = 407 $cm^3/g$, $M_w$ = 508 500 g/mol, $M_w/M_n$ = 2,4, Schmp. = 135°C. Laut $^{13}C$-NMR-Spektroskopie wurde das Ethylen überwiegend isoliert (statistisch) eingebaut.

Beispiel 14

Ein trockener 150 $dm^3$-Reaktor wurde mit Stickstoff gespült und bei 20°C mit 80 $dm^3$ eines entaromatisierten Benzinschnittes mit dem Siedebereich 100 bis 120°C gefüllt.
Dann wurde der Gasraum mit Propylen stickstofffrei gespült und 50 l flüssiges Propylen sowie 64 $cm^3$ toluolische Methylaluminoxanlösung (entsprechend 100 mmol Al, p = 19) zugegeben. Der Reaktorinhalt wurde auf 50°C aufgeheizt und durch Zudosierung von Wasserstoff wurde der Wasserstoffgehalt im Reaktorgasraum auf 0,2 % eingestellt und später während der Polymerisation dann durch Nachdosierung während der gesamten Polymerisationszeit konstant gehalten (Überprüfung on-Line durch Gaschromatographie).
14,9 mg rac-Dimethylsilandiylbis(2-methyl-4,5-benzoindenyl)zirkondichlorid wurden in 32 $cm^3$ toluolischer Methylaluminoxanlösung (50 mmol) gelöst und in den Reaktor gegeben.
Die Polymerisation erfolgte in einer ersten Stufe bei 65°C 6 h lang.
In einer 2. Stufe wurden dann bei 50°C 3 kg Ethylen schnell zugegeben und nach weiteren 4 Stunden Polymerisation bei dieser Temperatur wurde mit $CO_2$-Gas gestoppt. Es wurden 23,9 kg Blockcopolymerpulver erhalten. VZ = 398 $cm^3/g$, $M_w$ = 387 500 g/mol, $M_w/M_n$ = 4,5; MFI (230/5) = 14,5 dg/min.
Das Blockcopolymer enthielt 10,6 Gew.-% Ethylen. Die Fraktionierung ergab einen Gehalt von 26,9 Gew.-% Ethylen/Propylen-Kautschuk. Die Glastemperatur des Kautschuks war -48°C.

Beispiel 15

Es wurde verfahren wie in Beispiel 10, verwendet wurden jedoch 100 $Ndm^3$ Wasserstoff. Die Metallocenaktivität war 605 kg PP/g Metallocen x h, die VZ 17 $cm^3/g$ und der Schmelzpunkt 150°C.
Das Beispiel 15 zeigt, daß mit noch relativ geringen Mengen Wasserstoff sogar Wachse mit den erfindungsgemäßen Metallocenen herstellbar sind.

**Patentansprüche**

1. Verbindung der Formel I

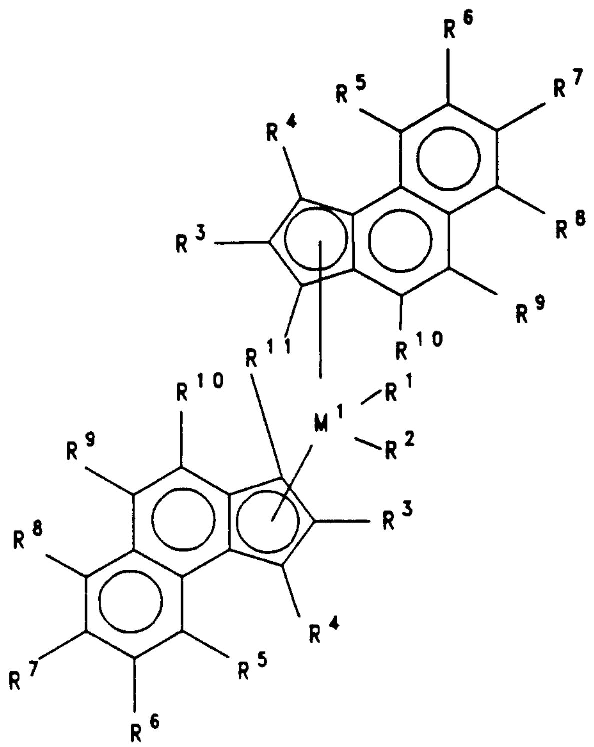

( I )

worin

| | |
|---|---|
| $M^1$ | ein Metall der Gruppe IVb, Vb oder VIb des Periodensystems ist, |
| $R^1$ und $R^2$ | gleich oder verschieden sind und ein Wasserstoffatom, eine $C_1$-$C_{10}$-Alkylgruppe, eine $C_1$-$C_{10}$-Alkoxygruppe, eine $C_6$-$C_{10}$-Arylgruppe, eine $C_6$-$C_{10}$-Aryloxygruppe, eine $C_2$-$C_{10}$-Alkenylgruppe, eine $C_7$-$C_{40}$-Arylalkylgruppe, eine $C_7$-$C_{40}$-Alkylarylgruppe, eine $C_8$-$C_{40}$-Arylalkenylgruppe, eine OH-Gruppe oder ein Halogenatom bedeuten, |
| die Reste $R^3$ | gleich oder verschieden sind und ein Wasserstoffatom, ein Halogenatom, eine $C_1$-$C_{10}$-Alkylgruppe, die halogeniert sein kann, eine $C_6$-$C_{10}$-Arylgruppe, einen $-NR_2$, $-SR$, $-OSiR_3$, $-SiR_3$ oder $PR_2$-Rest bedeuten, worin R ein Halogenatom, eine $C_1$-$C_{10}$-Alkylgruppe oder eine $C_6$-$C_{10}$-Arylgruppe ist, |
| $R^4$ bis $R^{10}$ | die für $R^3$ genannten Bedeutungen besitzen, oder benachbarte Reste $R^4$ bis $R^{10}$ mit den sie verbindenden Atomen einen aromatischen oder aliphatischen Ring bilden, |

$R^{11}$

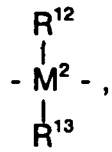 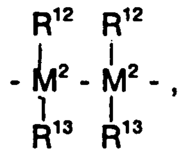 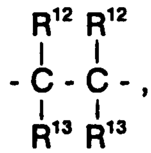 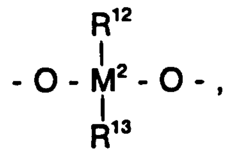

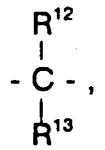 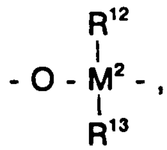 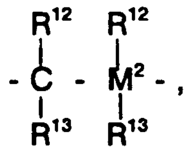

$=BR^{12}$, $=AlR^{12}$, -Ge-, -Sn-, -O-, -S-, =SO, $=SO_2$, $=NR^{12}$, =CO, $=PR^{12}$ oder $=P(O)R^{12}$ ist, wobei $R^{12}$ und $R^{13}$ gleich oder verschieden sind und ein Wasserstoffatom, ein Halogenatom, eine $C_1$-$C_{10}$-Alkylgruppe, $C_1$-$C_{10}$-Fluoralkylgruppe, eine $C_6$-$C_{10}$-Arylgruppe, eine $C_6$-$C_{10}$-Fluorarylgruppe, eine $C_1$-$C_{10}$-Alkoxygruppe, eine $C_2$-$C_{10}$-Alkenylgruppe, eine $C_7$-$C_{40}$-Arylalkylgruppe, eine $C_8$-$C_{40}$-Arylalkenylgruppe, eine $C_7$-$C_{40}$-Alkylarylgruppe bedeuten oder $R^{12}$ und $R^{13}$ jeweils mit den sie verbindenden Atomen einen Ring bilden und

$M^2$ Silizium, Germanium oder Zinn ist.

**2.** Verbindung der Formel I gemäß Anspruch 1, dadurch gekennzeichnet, daß in Formel I

$M^1$ Zirkonium oder Hafnium,
$R^1$ und $R^2$ gleich sind und eine $C_1$-$C_3$-Alkylgruppe oder ein Halogenatom, die Reste $R^3$ gleich sind und eine $C_1$-$C_4$-Alkylgruppe,
$R^4$ bis $R^{10}$ gleich oder verschieden sind und Wasserstoff oder eine $C_1$-$C_4$-Alkylgruppe und
$R^{11}$

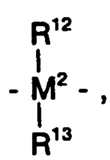 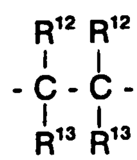 oder 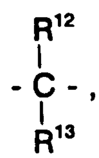

wobei $M^2$ für Silizium und $R^{12}$ und $R^{13}$ gleich oder verschieden sind und für eine $C_1$-$C_4$-Alkylgruppe oder eine $C_6$-$C_{10}$-Arylgruppe stehen, bedeuten.

**3.** Verbindung der Formel I gemäß Anspruch 1 oder 2, dadurch gekennzeichnet, daß in Formel I $R^4$ und $R^{10}$ Wasserstoff und $R^5$ bis $R^9$ gleich oder verschieden sind und Wasserstoff oder eine $C_1$-$C_4$-Alkylgruppe bedeuten.

**4.** Verbindung der Formel I gemäß den Ansprüche 1 bis 3, dadurch gekennzeichnet, daß in Formel I

$M^1$ Zirkonium,
$R^1$ und $R^2$ gleich sind und Chlor, die Reste $R^3$ gleich sind und eine $C_1$-$C_4$-Alkylgruppe, $R^4$ und $R^{10}$ Wasserstoff,
$R^5$ bis $R^9$ gleich oder verschieden sind und eine $C_1$-$C_4$-Alkylgruppe oder Wasserstoff und

$R^{11}$

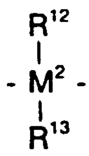 oder 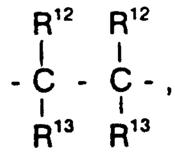

wobei $M^2$ Silizium

und $R^{12}$ und $R^{13}$ gleich oder verschieden sind und eine $C_1$-$C_4$-Alkylgruppe oder eine $C_6$-$C_{10}$-Arylgruppe bedeuten, bedeuten.

5. Verbindung der Formel I gemäß den Ansprüche 1 bis 4, dadurch gekennzeichnet, daß in Formel I $M^1$ Zirkonium ist, $R^1$ und $R^2$ Chlor bedeuten, $R^3$ Methyl ist, $R^4$ bis $R^{10}$ Wasserstoff bedeuten und

$R^{11}$

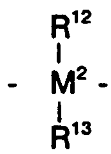

ist, wobei $M^2$ Silizium und $R^{12}$ und $R^{13}$

gleich oder verschieden sind und Methyl oder Phenyl bedeuten.

6. Dimethylsilandiylbis(2-methyl-4,5-benzoindenyl)zirkondichlorid.

7. Verfahren zur Herstellung einer Verbindung der Formel I gemäß den Ansprüche 1 bis 6, dadurch gekennzeichnet, daß man eine Verbindung der Formel IV

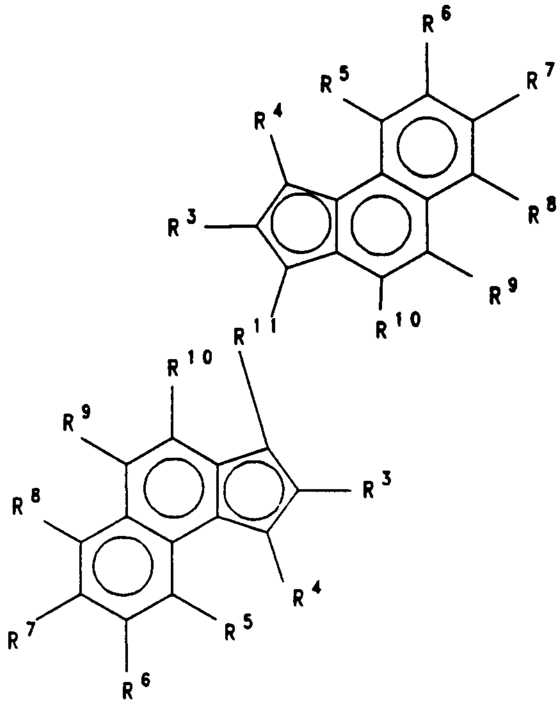

(IV)

wobei die Reste $R^3$ bis $R^{11}$ die in Formel I genannten Bedeutungen haben und $M^3$ ein Alkalimetall, bevorzugt Lithium, bedeutet, mit einer Verbindung der Formel V

$$M^1X_4 \qquad (V)$$

worin $M^1$ die in Formel I genannte Bedeutung besitzt und X ein Halogenatom, bevorzugt Chlor, bedeutet, umsetzt und das erhaltene Reaktionsprodukt gegebenenfalls derivatisiert.

8. Verfahren zur Herstellung eines Olefinpolymers durch Polymerisation oder Copolymerisation eines Olefins der Formel $R^a$-CH=CH-$R^b$, worin $R^a$ und $R^b$ gleich oder verschieden sind und ein Wasserstoffatom oder einen Kohlenwasserstoffrest mit 1 bis 14 C-Atomen bedeuten, oder $R^a$ und $R^b$ mit den sie verbindenden Atomen einen Ring bilden können, bei einer Temperatur von -60 bis 200°C, bei einem Druck von 0,5 bis 100 bar, in Lösung, in Suspension oder in der Gasphase, in Gegenwart eines Katalysators, welcher aus einem Metallocen als Übergangsmetallverbindung und einem Cokatalysator gebildet wird, dadurch gekennzeichnet, daß das Metallocen eine Verbindung der Formel I gemäß Ansprüche 1 bis 6 ist.

9. Verfahren gemäß Anspruch 8, dadurch gekennzeichnet, daß als Cokatalysator ein Aluminoxan der Formel (II)

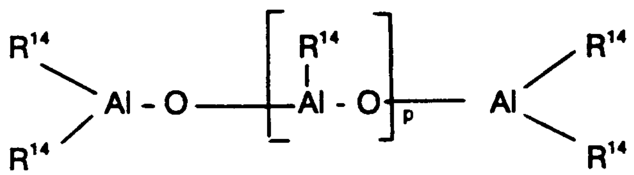

(II)

für den linearen Typ und/oder der Formel (III)

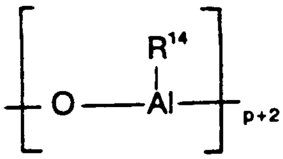

(III)

für den cyclischen Typ verwendet wird, wobei in den Formeln (II) und (III) die Reste $R^{14}$ gleich oder verschieden sind und eine $C_1$-$C_6$-Alkylgruppe, eine $C_6$-$C_{18}$-Arylgruppe, Benzyl oder Wasserstoff bedeuten und p eine ganze Zahl von 2 bis 50 ist.

**10.** Verfahren gemäß Anspruch 8 oder 9, dadurch gekennzeichnet, daß als Cokatalysator Methylaluminoxan verwendet wird.

**11.** Verfahren gemäß Anspruch 9 oder 10, dadurch gekennzeichnet, daß das Metallocen der Formel I vor dem Einsatz in der Polymerisationsreaktion mit einem Aluminoxan der Formel II und/oder III voraktiviert wird.

**12.** Verwendung eines Metallocens der Formel I gemäß den Ansprüche 1 bis 6 als Katalysatorkomponente bei der Olefinpolymerisation.

**13.** Verfahren gemäß den Ansprüche 8 bis 11, worin das Metallocen auf einen Träger aufgebracht ist.

**14.** Verfahren gemäß den Ansprüche 8 bis 11 und 13, worin das Metallocen vorpolymerisiert ist.

**15.** Katalysator, welcher aus einem Metallocen als Übergangsmetallverbindung und einem Cokatalysator gebildet wird, dadurch gekennzeichnet, daß das Metallocen eine Verbindung der Formel I gemäß den Ansprüche 1 bis 6 ist.

**16.** Katalysator gemäß Anspruch 15, dadurch gekennzeichnet, daß als Cokatalysator ein Aluminoxan der Formel (II)

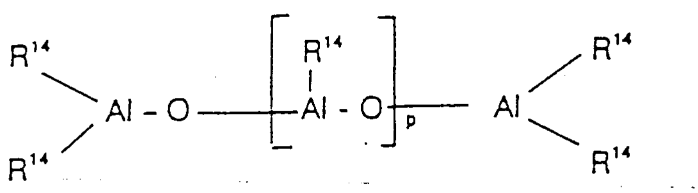

(II)

für den linearen Typ und/oder der Formel (III)

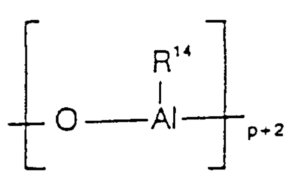

(III)

für den cyclischen Typ verwendet wird, wobei in den Formeln (II) und (III) die Reste $R^{14}$ gleich oder verschieden sind und eine $C_1$-$C_6$-Alkylgruppe, eine $C_6$-$C_{18}$-Arylgruppe, Benzyl oder Wasserstoff bedeuten und p eine ganze Zahl von 2 bis 50 ist.

**17.** Katalysator gemäß Anspruch 15 oder 16, dadurch gekennzeichnet, daß als Cokatalysator Methylaluminoxan verwendet wird.

**18.** Katalysator gemäß den Ansprüche 15 bis 17, wobei als Cokatalysator an Stelle oder neben eines Aluminoxans Verbindungen der Formel $R_xNH_{4-x}BR'_4$, $R_xPH_{4-x}BR'_4$, $R_3CBR'_4$ oder $BR'_3$ als verwendet werden, worin x eine Zahl

von 1 bis 4 bedeutet, die Reste R sind gleich oder verschieden und bedeuten $C_1$-$C_{10}$-Alkyl, $C_6$-$C_{18}$-Aryl oder 2 Reste R bilden zusammen mit dem sie verbindenden Atom einen Ring, und die Reste R' sind gleich oder verschieden und stehen für $C_6$-$C_{18}$-Aryl, das durch Alkyl, Haloalkyl oder Fluor substituiert sein kann.

**19.** Katalysator gemäß den Ansprüche 15 bis 18, worin das Metallocen auf einen Träger aufgebracht ist.

**20.** Katalysator gemäß den Ansprüche 15 bis 19, worin das Metallocen vorpolymerisiert ist.

## Claims

**1.** A compound of the formula I

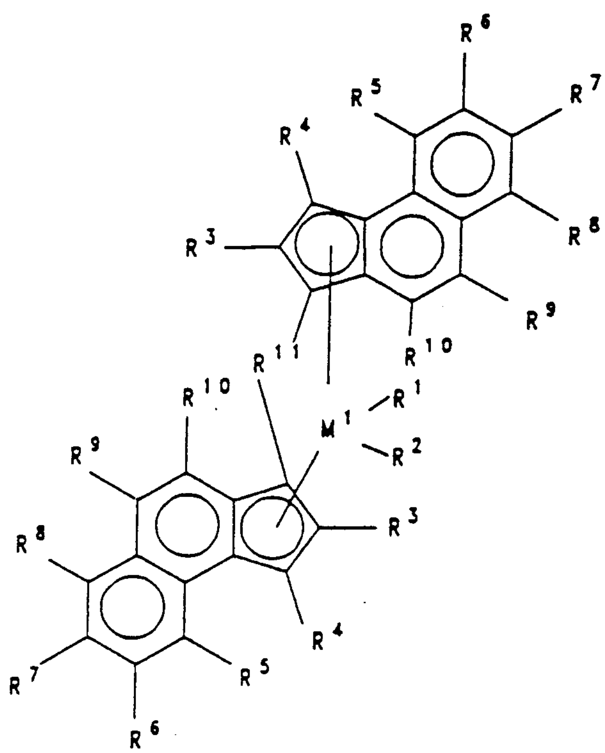

(I) (I)

in which

| | |
|---|---|
| $M^1$ | is a metal of group IVb, Vb or VIb of the periodic table, |
| $R^1$ and $R^2$ | are identical or different and are a hydrogen atom, a $C_1$-$C_{10}$-alkyl group, a $C_1$-$C_{10}$-alkoxy group, a $C_6$-$C_{10}$-aryl group, a $C_6$-$C_{10}$-aryloxy group, a $C_2$-$C_{10}$-alkenyl group, a $C_7$-$C_{40}$-arylalkyl group, a $C_7$-$C_{40}$-alkylaryl group, a $C_8$-$C_{40}$-arylalkenyl group, an OH group or a halogen atom, |
| the radicals $R^3$ | are identical or different and are a hydrogen atom, a halogen atom, a $C_1$-$C_{10}$-alkyl group, which can be halogenated, a $C_6$-$C_{10}$-aryl group or an $-NR_2$, $-SR$, $-OSiR_3$, $-SiR_3$ or $PR_2$ radical, in which R is a halogen atom, a $C_1$-$C_{10}$-alkyl group or a $C_6$-$C_{10}$-aryl group, |
| $R^4$ to $R^{10}$ | have the meanings given for $R^3$, or adjacent radicals $R^4$ to $R^{10}$, with the atoms joining them, form an aromatic or aliphatic ring, and |

$R^{11}$ is

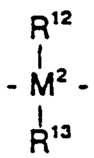, 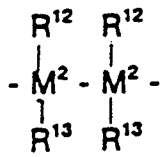, 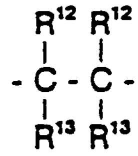, 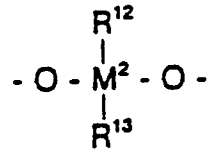,

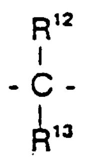, 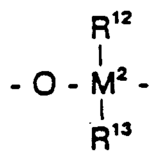, 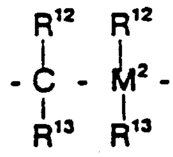,

$=BR^{12}$, $=AlR^{12}$, -Ge-, -Sn-, -O-, -S-, =SO, $=SO_2$, $=NR^{12}$, =CO, $=PR^{12}$ or $=P(O)R^{12}$ in which

$R^{12}$ and $R^{13}$ are identical or different and are a hydrogen atom, a halogen atom, a $C_1$-$C_{10}$-alkyl group, [lacuna] $C_1$-$C_{10}$-fluoroalkyl group, a $C_6$-$C_{10}$-aryl group, a $C_6$-$C_{10}$-fluoroaryl group, a $C_1$-$C_{10}$-alkoxy group, a $C_2$-$C_{10}$-alkenyl group, a $C_7$-$C_{40}$-arylalkyl group, a $C_8$-$C_{40}$-arylalkenyl group or a $C_7$-$C_{40}$-alkylaryl group, or $R^{12}$ and $R^{13}$, in each case with the atoms joining them, form a ring, and

$M^2$ is silicon, germanium or tin.

**2.** A compound of the formula I as claimed in claim 1, wherein, in formula I,

$M^1$ is zirconium or hafnium,
R1 and $R^2$ are identical and are a $C_1$-$C_3$-alkyl group or a halogen atom, the radicals $R^3$ are identical and are a $C_1$-$C_4$-alkyl group,
$R^4$ to $R^{10}$ are identical or different and are hydrogen or a $C_1$-$C_4$-alkyl group and
$R^{11}$ is

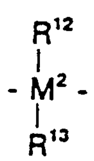, 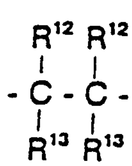 or 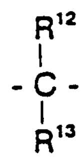,

in which $M^2$ is silicon and
$R^{12}$ and $R^{13}$ are identical or different and are a $C_1$-$C_4$-alkyl group or a $C_6$-$C_{10}$-aryl group.

**3.** A compound of the formula I as claimed in claim 1 or 2, wherein, in formula I, $R^4$ and $R^{10}$ are hydrogen and $R^5$ to $R^9$ are identical or different and are hydrogen or a $C_1$-$C_4$-alkyl group.

**4.** A compound of the formula I as claimed in one or more of claims 1 to 3, wherein, in formula I,

$M^1$ is zirconium,
$R^1$ and $R^2$ are identical and are chlorine, the radicals $R^3$ are identical and are a $C_1$-$C_4$-alkyl group, $R^4$ and $R^{10}$ are hydrogen,
$R^5$ to $R^9$ are identical or different and are a $C_1$-$C_4$-alkyl group or hydrogen and

$R^{11}$ is

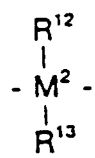

or

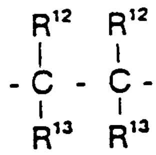

,

in which $M^2$ is silicon

and $R^{12}$ and $R^{13}$ are identical or different and are a $C_1$-$C_4$-alkyl group or a $C_6$-$C_{10}$-aryl group.

5. A compound of the formula I as claimed in one or more of claims 1 to 4, wherein, in formula I, $M^1$ is zirconium, $R^1$ and $R^2$ are chlorine, $R^3$ is methyl, $R^4$ to $R^{10}$ are hydrogen, and $R^{11}$ is

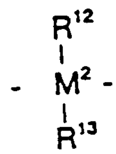

in which $M^2$ is silicon
and $R^{12}$ and $R^{13}$ are identical or different and are methyl or phenyl.

6. Dimethylsilanediylbis(2-methyl-4,5-benzoindenyl)zirconium dichloride.

7. A process for the preparation of a compound of the formula I as claimed in one or more of claims 1 to 6, which comprises reacting a compound of the formula IV

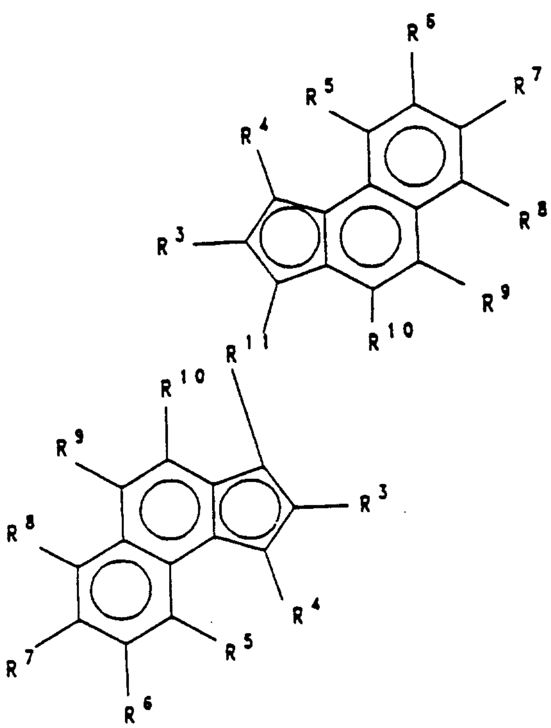

(IV)

in which the radicals $R^3$ to $R^{11}$ have the meanings given in formula I and $M^3$ is an alkali metal, preferably lithium, with a compound of the formula V

$$M^1X_4 \qquad (V)$$

in which $M^1$ has the meaning given in formula I and X is a halogen atom, preferably chlorine, and if appropriate derivatizing the resulting reaction product.

8. A process for the preparation of an olefin polymer by polymerization or copolymerization of an olefin of the formula $R^a$-CH=CH-$R^b$, in which $R^a$ and $R^b$ are identical or different and are a hydrogen atom or a hydrocarbon radical having 1 to 14 carbon atoms, or $R^a$ and $R^b$, with the atoms joining them, can form a ring, at a temperature of from -60 to 200°C, under a pressure of from 0.5 to 100 bar, in solution, in suspension or in the gas phase, in the presence of a catalyst which is formed from a metallocene, as the transition metal compound, and a cocatalyst, which comprises using a compound of the formula I as claimed in one or more of claims 1 to 6 as the metallocene.

9. The process as claimed in claim 8, wherein an aluminoxane of the formula (II)

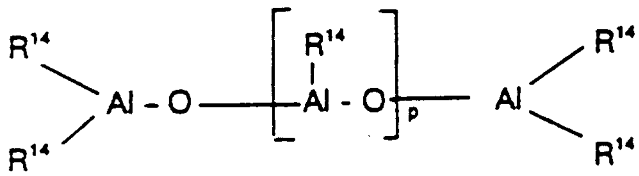

(II)

for the linear type, and/or of the formula (III)

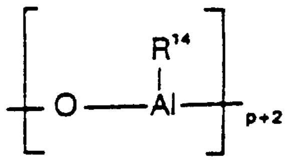

(III)

for the cyclic type, in which, in the formulae (II) and (III), the radicals $R^{14}$ are identical or different and are a $C_1$-$C_6$-alkyl group, a $C_6$-$C_{18}$-aryl group, benzyl or hydrogen and p is an integer from 2 to 50, is used as the cocatalyst.

10. The process as claimed in claim 8 or 9, wherein methylaluminoxane is used as the cocatalyst.

11. The process as claimed in claim 9 or 10, wherein the metallocene of the formula I is preactivated with an aluminoxane of the formula II and/or III before use in the polymerization reaction.

12. The use of a metallocene of the formula I as claimed in one or more of claims 1 to 6 as a catalyst component in olefin polymerization.

13. The process as claimed in one or more of claims 8 to 11, in which the metallocene is applied to a support.

14. The process as claimed in one or more of claims 8 to 11 and 13, in which the metallocene is prepolymerized.

15. A catalyst which is formed from a metallocene as transition metal compound and from a cocatalyst, wherein the metallocene is a compound of the formula I as claimed in one or more of Claims 1 to 6.

**16.** A catalyst as claimed in claim 15, wherein an aluminoxane of the formula (II)

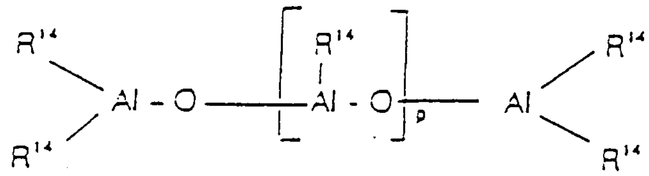

(II)

for the linear type, and/or of the formula (III)

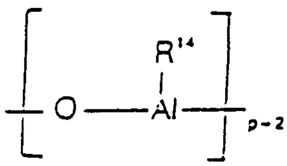

(III)

for the cyclic type, in which, in the formulae (II) and (III), the radicals $R^{14}$ are identical or different and are a $C_1$-$C_6$-alkyl group, a $C_6$-$C_{18}$-aryl group, benzyl or hydrogen and p is an integer from 2 to 50, is used as the cocatalyst.

**17.** A catalyst as claimed in claim 15 or 16, wherein methylaluminoxane is used as the cocatalyst.

**18.** A catalyst as claimed in one or more of claims 15 to 17, wherein the cocatalyst used, instead of or in addition to an aluminoxane, comprises compounds of the formula $R_xNH_{4-x}BR'_4$, $R_xPH_{4-x}BR'_4$, $R_3CBR'_4$ or $BR'_3$, in which x is a number from 1 to 4, the radicals R are identical or different and are $C_1$-$C_{10}$-alkyl or $C_6$-$C_{18}$-aryl, or 2 radicals R form a ring together with the atom which connects them, and the radicals R' are identical or different and are $C_6$-$C_{18}$-aryl which can be substituted by alkyl, haloalkyl or fluorine.

**19.** A catalyst as claimed in one or more of claims 15 to 18, in which the metallocene is applied to a support.

**20.** A catalyst as claimed in one or more of claims 15 to 19, in which the metallocene is prepolymerized.

## Revendications

**1.** Composé de formule I

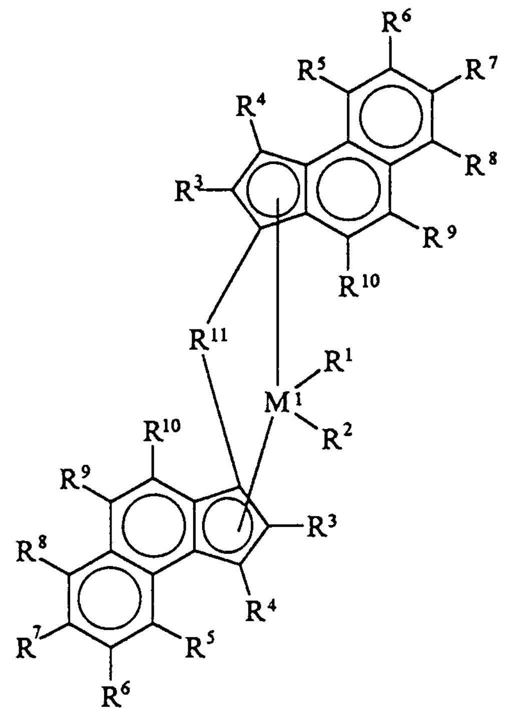

(I)

dans laquelle

| | |
|---|---|
| $M^1$ | est un métal du coupe IVb, Vb ou VIb du système périodique, |
| $R^1$ et $R^2$ | sont identiques ou différents et représentent un atome d'hydrogène, un groupe alkyle en $C_1$-$C_{10}$, un groupe alcoxy en $C_1$-$C_{10}$, un groupe aryle en $C_6$-$C_{10}$, un groupe aryloxy en $C_6$-$C_{10}$, un groupe alcényle en $C_2$-$C_{10}$, un groupe arylalkyle en $C_7$-$C_{40}$, un groupe alkylaryle en $C_7$-$C_{40}$, un groupe arylalcényle en $C_8$-$C_{40}$, un groupe OH ou un atome d'halogène, |
| les restes $R^3$ | sont identiques ou différents et représentent un atome d'hydrogène, un atome d'halogène, un groupe alkyle en $C_1$-$C_{10}$ qui peut être halogéné, un groupe aryle en $C_6$-$C_{10}$, un reste $-NR_2$, $-SR$, $-OSiR_3$, $-SiR_3$ ou $-PR_2$ dans lesquels R est un atome d'halogène, un groupe alkyle en $C_1$-$C_{10}$ ou un groupe aryle en $C_6$-$C_{10}$, |
| $R^4$ à $R^{10}$ | ont les significations données pour $R^3$, ou des restes $R^4$ à $R^{10}$ voisins forment avec les atomes qui les relient un noyau aromatique ou aliphatique, |
| $R^{11}$ | est |

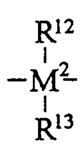 ,
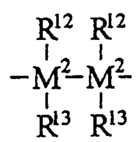 ,
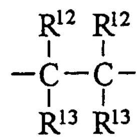 ,
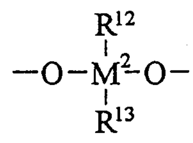 ,

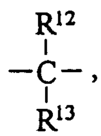, 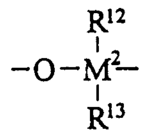, 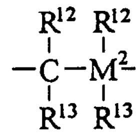,

$=BR^{12}$, $=AlR^{12}$, -Ge-, -Sn-, -O-, -S-, =SO, $=SO_2$, $=NR^{12}$, =CO, $=PR^{12}$ ou $=P(O)R^{12}$,
où
$R^{12}$ et $R^{13}$ sont identiques ou différents et représentent un atome d'hydrogène, un atome d'halogène, un groupe alkyle en $C_1$-$C_{10}$, un groupe fluoroalkyle en $C_1$-$C_{10}$, un groupe aryle en $C_6$-$C_{10}$, un groupe fluoroaryle en $C_6$-$C_{10}$, un groupe alcoxy en $C_1$-$C_{10}$, un groupe alcényle en $C_2$-$C_{10}$, un groupe arylalkyle $C_7$-$C_{40}$, un groupe arylalcényle en $C_8$-$C_{40}$, un groupe alkylaryle en $C_7$-$C_{40}$, ou bien $R^{12}$ et $R^{13}$ forment respectivement un cycle avec les atomes qui les relient et

$M^2$ est le silicium, le germanium ou l'étain.

**2.** Composé de formule I selon la revendication 1, caractérisé en ce que, dans la formule I,

$M^1$ est le zirconium ou l'hafnium,
$R^1$ et $R^2$ sont identiques et représentent un groupe alkyle en $C_1$-$C_3$ ou un atome d'halogène,
les restes $R^3$ sont identiques et représentent un groupe alkyle en $C_1$-$C_4$,
$R^4$ à $R^{10}$ sont identiques ou différents et représentent un atome d'hydrogène ou un groupe alkyle en $C_1$-$C_4$, et
$R^{11}$ est

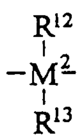, 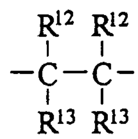 ou 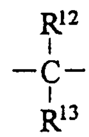,

où
$M^2$ est le silicium et

$R^{12}$ et $R^{13}$ sont identiques ou différents et représentent un groupe alkyle en $C_1$-$C_4$ ou aryle en $C_6$-$C_{10}$.

**3.** Composé de formule I selon la revendication 1 ou 2, caractérisé en ce que, dans la formule I, les restes $R^4$ et $R^{10}$ représentent l'hydrogène et les restes $R^5$ à $R^9$ sont identiques ou différents et représentent un atome d'hydrogène ou un groupe alkyle en $C_1$-$C_4$.

**4.** Composé de formule I selon l'une ou plusieurs des revendications 1 à 3, caractérisé en ce que, dans la formule I,

$M^1$ est le zirconium,
$R^1$ et $R^2$ sont identiques et représentent le chlore,
les restes $R^3$ sont identiques et représentent un groupe alkyle en $C_1$-$C_4$,
$R^4$ et $R^{10}$ sont des atomes d'hydrogène,
$R^5$ à $R^9$ sont identiques ou différents et représentent un atome d'hydrogène ou un groupe alkyle en $C_1$-$C_4$, et

$R^{11}$ est

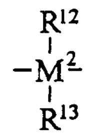 ou 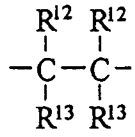 ,

où $M^2$ est le silicium et

$R^{12}$ et $R^{13}$ sont identiques ou différents et représentent un groupe alkyle en $C_1$-$C_4$ ou aryle en $C_6$-$C_{10}$.

**5.** Composé de formule I selon l'une ou plusieurs des revendications 1 à 4, caractérisé en ce que, dans la formule I, $M^1$ est le zirconium, $R^1$ et $R^2$ représentent le chlore, $R^3$ est un groupe méthyle, $R^4$ à $R^{10}$ sont des atomes d'hydrogène et $R^{11}$ est

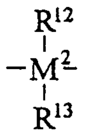 ,

où $M^2$ est le silicium et $R^{12}$ et $R^{13}$ sont identiques ou différents et représentent un groupe méthyle ou phényle.

**6.** Dichlorure de diméthylsilanediylbis(2-méthyl-4,5-benzoindényl)zirconium.

**7.** Procédé de préparation d'un composé de formule I selon l'une ou plusieurs des revendications 1 à 6, caractérisé en ce que l'on fait réagir un composé de formule IV

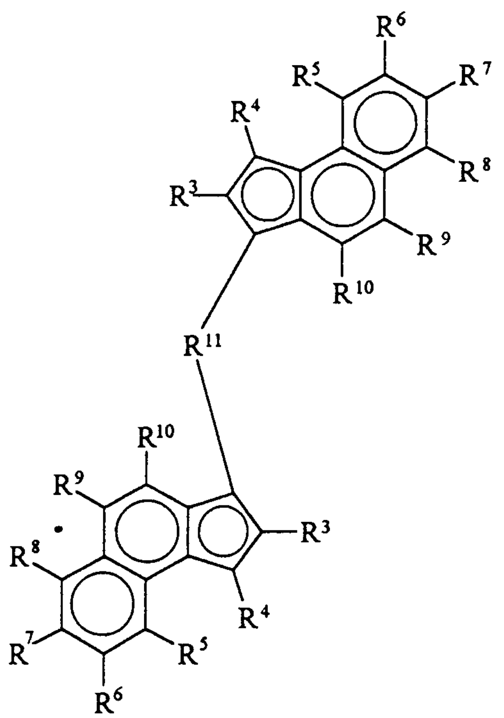

(IV)

dans laquelle les restes $R^3$ à $R^{11}$ ont la signification donnée pour la formule I et $M^3$ est un métal alcalin, de préférence le lithium, avec un composé de formule V

$$M^1X_4 \qquad (V)$$

dans laquelle $M^1$ a la signification donnée pour la formule I et X est un atome d'halogène, de préférence de chlore, et on transforme éventuellement le produit de réaction obtenu en un de ses dérivés.

8. Procédé de préparation d'un polymère oléfinique par polymérisation ou copolymérisation d'une oléfine de formule $R^a$-CH=CH-$R^b$, dans laquelle $R^a$ et $R^b$ sont identiques ou différents et représentent un atome d'hydrogène ou un reste hydrocarboné de 1 à 14 atomes de carbone, ou bien $R^a$ et $R^b$ peuvent former un cycle avec les atomes qui les relient, à une température de -60 à 200°C, sous une pression de 0,5 à 100 bars, en solution, en suspension ou en phase gazeuse, en présence d'un catalyseur formé à partir d'un métallocène en tant que composé d'un métal de transition et d'un co-catalyseur, caractérisé en ce que le métallocène est un composé de formule I selon l'un ou plusieurs des revendications 1 à 6.

9. Procédé selon la revendication 8, caractérisé en ce que l'on utilise comme co-catalyseur un aluminoxane de formule (II)

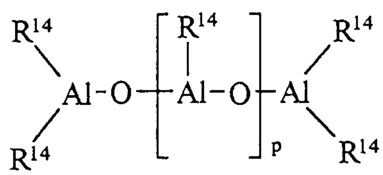

(II)

en ce qui concerne le type linéaire, et/ou de formule III

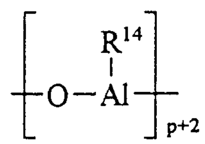

(III)

en ce qui concerne le type cyclique, où, dans les formules (II) et (III), les restes $R^{14}$ sont identiques ou différents et représentent un groupe alkyle en $C_1$-$C_6$, un groupe aryle en $C_1$-$C_{18}$, un groupe benzyle ou un atome d'hydrogène et p est un nombre entier de 2 à 50.

10. Procédé selon la revendication 8 ou 9, caractérisé en ce que l'on utilise un méthylaluminoxane comme co-catalyseur.

11. Procédé selon la revendication 9 ou 10, caractérisé en ce que l'on active au préalable le métallocène de formule I avec un aluminoxane de formule II et/ou III avant de l'introduire dans la réaction de polymérisation.

12. Utilisation d'un métallocène de formule I selon l'une ou plusieurs des revendications 1 à 6 comme constituant d'un catalyseur pour la polymérisation des oléfines.

13. Procédé selon l'une ou plusieurs des revendications 8 à 11, dans lequel le métallocène est appliqué sur un support.

14. Procédé selon l'une ou plusieurs des revendications 8 à 11 et 13, dans lequel le métallocène est prépolymérisé.

15. Catalyseur constitué d'un métallocène en tant que composé d'un métal de transition et d'un co-catalyseur, caractérisé en ce que le métallocène est un composé de formule I selon l'une ou plusieurs des revendications 1 à 6.

16. Catalyseur selon la revendication 15, caractérisé en ce que l'on utilise comme co-catalyseur un aluminoxane de formule (II)

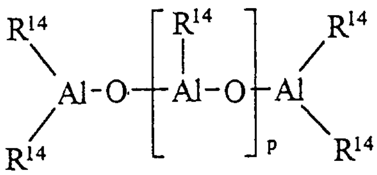

(II)

en ce qui concerne le type linéaire, et/ou de formule III

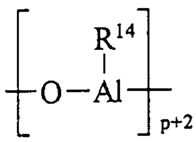

(III)

en ce qui concerne le type cyclique, où, dans les formules (II) et (III), les restes $R^{14}$ sont identiques ou différents et représentent un groupe alkyle en $C_1$-$C_6$, un groupe aryle en $C_6$-$C_{18}$, un groupe benzyle ou un atome d'hydrogène et p est un nombre entier de 2 à 50.

**17.** Catalyseur selon la revendication 15 ou 16, caractérisé en ce que l'on utilise un méthylaluminoxane comme co-catalyseur.

**18.** Catalyseur selon l'une ou plusieurs des revendications 15 à 17, dans lequel on utilise comme co-catalyseur, à la place ou en plus d'un aluminoxane, des composés de formule $R_xNH_{4-x}BR'_4$, $R_xPH_{4-x}BR'_4$, $R_3CBR'_4$ ou $BR'_3$, où x représente un nombre de 1 à 4, les restes R sont identiques ou différents et représentent un reste alkyle en $C_1$-$C_{10}$ ou aryle en $C_6$-$C_{18}$, ou deux restes R forment ensemble un cycle avec l'atome qui les relie, et les restes R' sont identiques ou différents et représentent un reste aryle en $C_6$-$C_{18}$ qui peut être substitué par alkyle, halogénoalkyle ou fluoro.

**19.** Catalyseur selon l'une ou plusieurs des revendications 15 à 18, dans lequel le métallocène est appliqué sur un support.

**20.** Catalyseur selon l'une ou plusieurs des revendications 15 à 19, dans lequel le métallocène est prépolymérisé.